# EUROPÄISCHE PATENTSCHRIFT

(11) **EP 3 692 085 B1**
(45) Veröffentlichungstag und Bekanntmachung des Hinweises auf die Patenterteilung: **22.06.2022**
(21) Anmeldenummer: 18778930.0
(22) Anmeldetag: 02.10.2018
(51) Int. Cl.: C08G 18/48, C08G 18/12, C08G 18/34, C08G 18/40

(54) **VISKO-ELASTISCHES ELEMENT AUF BASIS EINES POLYURETHAN-SCHAUMS**
VISCO-ELASTIC ELEMENT BASED ON A POLYURETHANE FOAM
ÉLÉMENT VISCOÉLASTIQUE À LA BASE D'UNE MOUSSE POLYURÉTHANE

(30) Priorität: 07.10.2017 EP 17195351
(43) Veröffentlichungstag der Anmeldung: 12.08.2020
(73) Patentinhaber: Covestro Deutschland AG, 51373 Leverkusen (DE)
(72) Erfinder: WEISER, Marc-Stephan, 51515 Kürten-Dürscheid (DE); PLUG, Sascha, 51375 Leverkusen (DE); DÖRR, Sebastian, 40593 Düsseldorf (DE); MOTLEY, Jeffrey, Staffordshire DE13 8DT (GB)
(74) Vertreter: Levpat
(86) Internationale Anmeldenummer: PCT/EP2018/076852
(87) Internationale Veröffentlichungsnummer: WO 2019/068732

(56) Entgegenhaltungen:
- US-A1- 2016 200 855

## Beschreibung

Die vorliegende Erfindung betrifft ein visko-elastisches Element beinhaltend einen Polyurethan-Schaum, wobei der Polyurethan-Schaum erhältlich ist durch Reaktion von mindestens einem isocyanatfunktionellen Prepolymer (V1) in Anwesenheit von einer speziellen Polyurethanharnstoff-Dispersion (V2), wobei die Reaktion des Prepolymers (V1) in Anwesenheit des Polyurethanharnstoff (V2) mit einem isocyanatreaktive-Gruppen enthaltenden Medium stattfindet. Die Erfindung betrifft weiterhin ein Verfahren zur Herstellung des visko-elastischen Elements und dessen Verwendung.

Polyurethan-Schäume sind bekannt als Wundauflagen für medizinische Anwendungen. Diese Schäume weisen bevorzugt eine hohe Absorptions- und Retentionsrate für Wasser auf, wie in EP 2 143 744 A1 gezeigt.

Die nach EP 2 143 744 A1 erhaltenen Schäume sind zwar aus einem medizinisch zugelassenen Rohmaterial gefertigt, jedoch sind sie für eine Anwendung als Schallisolierung/ Ohrstöpsel nicht geeignet, insbesondere zeigen sie ein zu schnelles Rückstellvermögen bei Kompression. Für eine Anwendung als Schallisolierung/ Ohrstöpsel ist es wichtig, dass der Schaum nach Zusammendrücken nur langsam in seine Form zurück kehrt bzw. sich anderen räumlichen Gegebenheiten wie dem Innenohr anpassen kann.

Eine Aufgabe der vorliegenden Erfindung ist es, mindestens einen Nachteil des Standes der Technik mindestens zu einem Teil zu überwinden.

Eine weitere Aufgabe der vorliegenden Erfindung ist es, ein visko-elastisches Element herzustellen, beispielsweise auf der Basis medizinisch zugelassener Rohstoffe, wie die in EP 2 143 744 A1 beschriebenen Materialien, und diese dabei so zu modifizieren, dass ein im Vergleich dazu langsameres Rückstellverhalten erreicht wird.

Zusätzlich ist es eine Aufgabe der vorliegenden Erfindung ein visko-elastisches Element bereitzustellen, das eine optimierte Rückstellzeit aufweist.

Eine weitere Aufgabe der vorliegenden Erfindung ist es, einen möglichst hohen Tragekomfort eines Gehörschutzstöpsels, in Form eines visko-elastischen Elements, beispielsweise im Ohr eines Benutzers zu erzielen.

Weiterhin ist eine Aufgabe der vorliegenden Erfindung, ein Verfahren zur Herstellung eines visko-elastischen Elements bereitzustellen, das eine Herstellung des Elements in wenigen Schritten möglichst resourcensparend ermöglicht. Es ist weiterhin eine Aufgabe der vorliegenden Erfindung, ein Verfahren zur Herstellung eines visko-elastischen Elements bereitzustellen, dass bei Raumtemperatur und Normaldruck durchführbar ist.

Weiterhin ist eine Aufgabe der vorliegenden Erfindung, ein visko-elastisches Element bereitzustellen, dass zur Anwendung in Gehörgängen von Benutzern, insbesondere zur Geräuschminimierung geeignet ist.

Es ist weiterhin eine Aufgabe der vorliegenden Erfindung, ein Kit bereitzustellen, das eine einfache Möglichkeit der Herstellung des visko-elastischen Elements ermöglicht.

Ein erster Gegenstand der Erfindung betrifft ein visko-elastisches Element, das im Folgenden auch als Produkt bezeichnet wird, beinhaltend einen Polyurethan-Schaum, erhältlich durch Reaktion von mindestens den Komponenten
- (V1): einem isocyanatfunktionellen Prepolymer, erhältlich durch Umsetzung von mindestens den Komponenten
V1A) aliphatischen Diisocyanaten mit
V1B) di- bis hexafunktionellen Polyalkylenoxiden mit einem Ethylenoxidanteil von 50 bis 100 mol%, bezogen auf die Gesamtmenge der enthaltenen Oxyalkylengruppen,
in Anwesenheit von
- (V2): einer Polyurethanharnstoff-Dispersion, wobei der Polyurethanharnstoff erhältlich ist durch Umsetzung von wenigstens
V2A) einer aliphatischen Polyisocyanat-Komponente mit einer mittleren Isocyanatfunktionalität von ≥ 1,8 und ≤ 2,6,
V2B) einer polymeren Polyether-Polyol-Komponente,
V2C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
V2D) gegebenenfalls weiteren hydrophilierenden Komponenten, die unterschiedlich sind von C2),
V2E) gegebenenfalls hydroxyfunktionellen Verbindungen mit einem Molekulargewicht von 62 bis 399 g/mol,
V2F) gegebenenfalls mindestens einem weiteren polymeren Polyol, welches unterschiedlich ist von V2B),
V2G) einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
V2H) gegebenenfalls einer aliphatischen Polyisocyanat-Komponente mit einer mittleren Isocyanatfunktionalität von ≥ 2,6 und ≤ 4,
wobei die Reaktion des Prepolymers (V1) in Anwesenheit des Polyurethanharnstoff (V2) mit einem isocyanatreaktive-Gruppen enthaltenden Medium stattfindet.

Als Visko-Elastizität bezeichnet man ein teilweise elastisches, teilweise viskoses Materialverhalten. Visko-elastische Stoffe vereinigen also Merkmale von Flüssigkeiten und Festkörpern in sich. Der Effekt ist zeit-, temperatur- und frequenzabhängig und tritt bei polymeren Schmelzen und Festkörpern wie z. B. Kunststoffen aber auch anderen Materialien auf.

Alle Flüssigkeiten und Feststoffe können wie visko-elastische Materialien betrachtet werden, wenn ihr Speicher- und Verlustmodul, G' und G", beziehungsweise ihr Verlustfaktor tan δ = G"/G' angegeben werden kann. Bei ideal-viskosen Flüssigkeiten (Newton'sches Fluid) ist der Speichermodul sehr klein gegenüber dem Verlustmodul, bei ideal-elastischen Festkörpern, die dem Hookschen Gesetz gehorchen, ist der Verlustmodul sehr klein gegenüber dem Speichermodul. Visko-elastische Materialien weisen sowohl einen messbaren Speichermodul als auch einen messbaren Verlustmodul auf. Falls der Speichermodul größer ist als der Verlustmodul, spricht man von Feststoffen, andernfalls von Flüssigkeiten. Bevorzugt ist das visko-elastische Element ein Feststoff, der sowohl einen messbaren Speichermodul als auch einen messbaren Verlustmodul aufweist. Bevorzugt weist das visko-elastische Element ein größeres Speichermodul als Verlustmodul auf. Bevorzugt weist das visko-elastische Element ein Speichermodul auf, das in einem Bereich von 1,1 bis 7 mal, oder bevorzugt in einem Bereich von 1,5 bis 6,5 mal, oder bevorzugt in einem Bereich von 2 bis 6 mal so groß ist wie das Verlustmodul.

Der Verlustfaktor ist also ein Maß für die Dämpfung eines visko-elastischen Körpers oder eines visko-elastischen Elements. Der Verlustfaktor tan δ des erfindungsgemäßen Elements liegt bei einer Druck- oder Zugverformung in Wirkungsrichtung bevorzugt bei 0,15 bis 0,50, oder bevorzugt bei 0,16 bis 0,45, oder bevorzugt bei 0,17 bis 0,40, oder bevorzugt bei 0,17 bis 0,35 gemessen nach DIN EN ISO 6721-1 Bestimmung dynmaisch-mechanischer Eigenschaften (unter Verwendung der Schwingungsart I, nicht resonant, Modultyp Gₜₒ, Schwingungsart IV und Teil 2).

Es konnte überraschend gezeigt werden, dass basierend auf den genannten Komponenten V1) und V2) Produkte erhalten werden konnten, die auf der einen Seite eine angenehme, weiche Haptik und auf der anderen Seite visko-elastische Eigenschaften aufweisen. Die visko-elastischen Eigenschaften bewirken, dass das visko-elastische Element bei Verformung keine spontane sondern eine retardierte Rückstellung in seine ursprüngliche Form aufweist, wenn die Kraft zur Verformung abgestellt wird. Diesen als visko-elastisch bezeichneten Effekt kann man sich in unterschiedlichsten Verwendungen des erfindungsgemäßen Elements zu Nutze machen. So kann dieser dazu dienen Kissen, Matratzen, Sitze oder ähnliche Strukturen sehr weich erscheinen zu lassen, aber insbesondere, um nach dem Zusammenpressen und in eine Kavität gesteckt sich der Geometrie der Kavität extrem gut anzupassen ohne Druck auf die Kavitätswandungen auszuüben.

Als Komponente V1A) kann jedes Diisocyanat verwendet werden, dass der Fachmann hierfür auswählen würde. Bevorzugt weisen die aliphatischen Diisocyanate V1A) eine Molmasse von 140 bis 278 g/mol auf.

Beispiele für geeignete aliphatische Diisocyanate V1A) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat, Isophorondiisocyanat, Butylendiisocyanat und Bis(isocyanatocyclohexyl)methan bevorzugt sind. Besonders bevorzugt sind Butylendiisocyanat, Hexamethylendiisocyanat, Isophorondiisocyanat, ganz besonders bevorzugt Hexamethylendiisocyanat und Isophorondiisocyanat.

Als Komponente V1B) kann jedes Polyalkylenoxide verwendet werden, dass der Fachmann hierfür auswählen würde.

Vorteilhaft ist auch, wenn die di- bis hexafunktionellen Polyalkylenoxide V1B) eine OH-Zahl von 22,5 bis 112 aufweisen.

Die Polyalkylenoxide weisen einen Ethylenoxidgehalt von 50 bis 100 mol%, bevorzugt von 55 bis 95 mol%, oder bevorzugt von 60 bis 80 mol%, bezogen auf die Gesamtmenge der enthaltenen Oxyalkylengruppen, auf.

Die Polyalkylenoxide besitzen typischerweise zahlenmittlere Molekulargewichte von 1000 bis 15000 g/mol, bevorzugt von 1500 bis 12000 g/mol, oder bevorzugt von 2000 bis 10000 g/mol, oder bevorzugt von 2500 bis 9000 g/mol, oder bevorzugt von 3000 bis 8000 g/mol.

Ferner besitzen die Polyalkylenoxide der Komponente V1B) bevorzugt OH-Funktionalitäten von 2 bis 6, weiter bevorzugt von 3 bis 6, besonders bevorzugt von 3 bis 4.

Geeignete Polyalkylenoxide sind beispielsweise Copolymere aus Ethylenoxid und Propylenoxid.

Besonders bevorzugt ist auch, wenn die Polyalkylenoxide auf Basis von Polyolen oder Aminen gestartet sind. Geeignete Starter sind Glycerin, Trimethylolpropan (TMP), Sorbit, Pentaerythrit, Triethanolamin, Ammoniak oder Ethylendiamin.

Bei der Herstellung der isocyanatfunktionellen Prepolymere V1) wird das Verhältnis der Polyalkylenoxide V1B) zu den niedermolekularen, aliphatischen Diisocyanaten V1A) typischerweise so eingestellt, dass auf 1 Mol OH-Gruppen des Polyalkylenoxide V1B) 2 bis 20 Mol, bevorzugt mit 2 bis 10 Mol und besonders bevorzugt 5 bis 10 Mol NCO-Gruppen des niedermolekularen, aliphatischen Diisocyanats V1A) kommen.

Die Herstellung der Prepolymeren erfolgt bevorzugt in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen.

Die Umsetzung der Polyalkylenoxide V1B) mit den Diisocyanaten V1A) zum Prepolymer V1) erfolgt typischerweise bei 25 bis 140 °C, bevorzugt bei 60 bis 100 °C.

Wurde bei der Umsetzung ein Überschuss an Diisocyanat V1A) eingesetzt, wird der nicht umgesetzte Rest anschließend bevorzugt durch Dünnschichtdestillation entfernt.

Vor, während und nach der Umsetzung der Diisocyanate V1A) mit den Polyalkylenoxiden V1B) oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien wie Di-*tert-*butylkresol oder Tocopherol zugesetzt werden.

Bevorzugt weisen die eingesetzten Prepolymere V1) einen Restmonomergehalt von unter 0,5 Gew.-%,bevorzugt von unter 0,4 Gew.-%, oder bevorzugt von weniger als 0,3 Gew.-%, bezogen auf das Gesamtgewicht des Prepolymer auf. Dieser Gehalt kann durch entsprechend gewählte Einsatzmengen der Diisocyanate V1A) und der Polyalkylenoxide V1B) erreicht werden. Bevorzugt ist jedoch der Einsatz des Diisocyanats V1A) im Überschuss und mit anschließender, bevorzugt destillativer, Abtrennung nicht umgesetzter Monomere.

Der NCO-Gehalt der isocyanatfunktionellen Prepolymere V1) beträgt bevorzugt 1,5 bis 4,5 Gew.-%, besonders bevorzugt 1,5 bis 3,5 Gew.-% und ganz besonders bevorzugt 1,5 bis 3,0 Gew.-%, bezogen auf das Gesamtgewicht des Prepolymer.

Eine wässrige Dispersion im Sinne der vorliegenden Erfindung ist ein heterogenes Gemisch eines partikulären Feststoffs (disperse Phase) in einer Wasser enthaltenden flüssigen Phase (Dispergiermedium). Bevorzugt ist eine Dispersion, bei der der mittlere Durchmesser der dispersen Phase bestimmt durch Laser-Korrelationsspektroskopie 1 bis 1000 nm, bevorzugt 2 bis 800 nm, weiter bevorzugt 5 bis 500 nm und besonders bevorzugt 20 bis 400 nm beträgt. Ebenfalls bevorzugt ist, wenn das Dispergiermedium ausschließlich aus Wasser besteht.

Polyurethanharnstoffe im Sinne der Erfindung sind polymere Verbindungen, die mindestens zwei, bevorzugt mindestens drei Urethan-Gruppen-haltige Wiederholungseinheiten aufweisen:

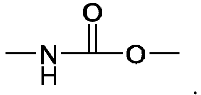

Erfindungsgemäß weisen die Polyurethanharnstoffe herstellungsbedingt auch Harnstoff-Gruppen-haltige Wiederholungseinheiten auf, 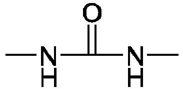 wie sie insbesondere bei der Umsetzung von isocyanatterminierten Präpolymeren mit aminofunktionellen Verbindungen gebildet werden.

Unter ionogenen Gruppen werden im Sinne dieser Erfindung solche funktionellen Gruppen verstanden, die in der Lage sind, beispielsweise durch Neutralisation mit einer Base, ionische Gruppen zu bilden.

Die Komponente V2A) kann jedes Polyisocyanat sein, das der Fachmann hierfür verwenden würde. Als Komponente V2A) bevorzugt geeignete Polyisocyanate sind insbesondere die dem Fachmann an sich bekannten aliphatischen Polyisocyanate mit einer mittleren Isocyanatfunktionalität von ≥ 1,8 und ≤ 2,6, bevorzugt von ≥ 1,9 und ≤ 2,5. Der Begriff aliphatisch umfasst dabei auch cycloaliphatische und/oder araliphatische Polyisocyanate.

Unter der mittleren Isocyanatfunktionalität wird dabei die durchschnittliche Anzahl an Isocyanatgruppen pro Molekül verstanden.

Bevorzugte Polyisocyanate sind solche des Molekulargewichtsbereichs von 140 bis 336 g/mol. Besonders bevorzugt sind diese ausgewählt aus der Gruppe bestehend aus. 1,4-Diisocyanatobutan (BDI), 1,5-Pentandiisocyanat, (PDI) 1,6-Diisocyanatohexan (HDI), 1,3-Bis(isocyanatomethyl)benzol (1,3-Xylylendiisocyanat, XDI), 1,4-Bis(isocyanatomethyl)benzol (1,4-Xylylendiisocyanat, XDI), 1,3-Bis(1-isocyanato-1-methyl-ethyl)benzol (TMXDI), 1,4-Bis(1-isocyanato-1-methyl-ethyl)benzol (TMXDI), 4-Isocyanatomethyl-1,8-octandiisocyanat (Trisisocyanatononan (TIN)), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan sowie die cycloaliphatischen Diisocyanate 1,3- bzw. 1,4-Diisocyanatocyclohexan, 1,4-Diisocyanato-3,3,5-trimethylcyclohexan, 1,3-Diisocyanato-2(4)-methylcyclohexan, 1-Isocyanato-3,3,5-trimethyl-5-isocyanatomethylcyclohexan (Isophorondiisocyanat, IPDI), 1-Isocyanato-1-methyl-4(3)isocyanato-methylcyclohexan, 1,8-Diisocyanato-p-menthan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetramethyl-1,1'-bi(cyclohexyl), 4,4'- und/oder 2,4'-Diisocyanatodicyclohexylmethan, 4,4'-Diisocyanato-3,3'-dimethyldicyclohexylmethan, 4,4'-Diisocyanato-3,3',5,5'-tetramethyldicyclohexylmethan, 1,3-Diisocyanatoadamantan, und 1,3-Dimethyl-5,7-diisocyanatoadamantan oder beliebige Gemische solcher Isocyanate. Ganz besonders bevorzugt sind die Polyisocyanate ausgewählt aus 1,4-Butylendiisocyanat, 1,5-Pentamethylendiisocyanat (PDI), 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)-methane oder deren Mischungen beliebigen Isomerengehalts (H12-MDI), 1,4-Cyclohexylendiisocyanat, 4-Isocyanatomethyl-1,8-octandiisocyanat (Nonantriisocyanat) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate, die eine mittlere Isocyanatfunktionalität ≥ 2 und ≤ 2,6 aufweisen, mit Uretdion-, Isocyanurat-, Urethan-, Allophanat-, Biuret-, Iminooxadiazindion- oder Oxadiazintrionstruktur sowie Mischungen aus diesen und/oder den oben stehenden anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von ≥ 1,8 und ≤ 2,6 und besonders bevorzugt ≥ 2,0 und ≤ 2,4.

Besonders bevorzugt enthält die organische Polyisocyanat-Komponente V2A) ein aliphatisches oder cycloaliphatisches Polyisocyanat ausgewählt aus HDI, IPDI und/oder H12-MDI oder deren Modifikationsprodukten, ganz besonders bevorzugt ausgewählt aus HDI und/oder IPDI.

In einer insbesondere bevorzugten Variante liegen als Komponente V2A) IPDI und HDI im Gemisch vor.

Das Gewichtsverhältnis von IPDI:HDI liegt dabei bevorzugt im Bereich von 1,05 bis 10, besonders bevorzugt im Bereich von 1,1 bis 5, und ganz besonders bevorzugt im Bereich von 1,1 bis 1,5.

Bevorzugt wird zur Herstellung des erfindungsgemäß verwendeten Polyurethanharnstoffs ≥ 5 und ≤ 40 Gew.-% der Komponente V2A) und besonders bevorzugt ≥ 10 und ≤ 35 Gew.-% der Komponente V2A), jeweils bezogen auf die Gesamtmasse des Polyurethanharnstoffs, eingesetzt.

Bevorzugt wird zur Herstellung des Polyurethanharnstoffs auch die Komponente V2H), eine aliphatische Polyisocyanat-Komponente mit einer mittleren Isocyanatfunktionalität (mittlere Anzahl Isocyanatgruppen pro Molekül) von ≥ 2,6 und ≤ 4, vorzugsweise ≥ 2,8 und ≤ 3,8 eingesetzt. Die Komponente V2H) wird dabei bevorzugt im Gemisch mit Komponente V2A) eingesetzt.

Als Komponente V2H) besonders geeignet sind oligomere Diisocyanate, die eine Funktionalität ≥ 2,6 und ≤ 4, vorzugsweise ≥ 2,8 und ≤ 3,8 aufweisen, mit Isocyanurat-, Urethan-, Allophanat-, Biuret-, Iminooxadiazindion- oder Oxadiazintrionstruktur. Ganz besonders bevorzugt enthält V2H) Isocyanuratstrukturen.

Besonders bevorzugt besteht die organische Polyisocyanat-Komponente V2H) aus einem aliphatischen oder cycloaliphatischen Polyisocyanat-Oligomer basierend auf HDI, IPDI und/oder H12-MDI, ganz besonders bevorzugt basierend auf HDI.

Das Molverhältnis der NCO-Gruppen aus Komponente V2A) zu Komponente V2H) beträgt dabei bevorzugt 100:0,5 bis 100: 50; besonders bevorzugt 100: 2 bis 100: 15 und ganz besonders bevorzugt 100:3 bis 100: 8.

Bevorzugt wird zur Herstellung des erfindungsgemäß verwendeten Polyurethanharnstoffs ≥ 0 und ≤ 10 Gew.-% der Komponente V2H) und besonders bevorzugt ≥ 0,1 und ≤ 3 Gew.-% der Komponente V2H), jeweils bezogen auf die Gesamtmasse des Polyurethanharnstoffs, eingesetzt.

Die erfindungsgemäß als Komponente V2B) eingesetzten polymeren Polyether-Polyole weisen bevorzugt zahlenmittlere Molekulargewichte von ≥ 500 und ≤ 8000 g/mol, bestimmt durch Gelpermeationschromatographie gegenüber Polystyrol-Standard in Tetrahydrofuran bei 23°C, bevorzugter ≥ 400 und ≤ 6000 g/mol und besonders bevorzugt von ≥ 600 und ≤ 3000 g/mol und/oder OH-Funktionalitäten von bevorzugt ≥ 1,5 und ≤ 6, bevorzugter ≥ 1,8 und ≤ 3, besonders bevorzugt von ≥ 1,9 und ≤ 2,1 auf.

Der Ausdruck "polymere" Polyether-Polyole bedeutet hier insbesondere, dass die genannten Polyole mindestens drei, bevorzugter mindestens vier miteinander verbundene Wiederholungseinheiten aufweisen.

Das zahlenmittlere Molekulargewicht wird im Rahmen dieser Anmeldung bestimmt durch Gelpermeationschromatographie (GPC) in Tetrahydrofuran bei 23°C, außer wenn dies anders beschrieben wird. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2×PSS SDV linear M, 8×300 mm, 5 µm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.

Geeignete Polyetherpolyole sind beispielsweise die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

In einer bevorzugten Ausführungsform des Produktes enthält die Komponente V2B) Poly(tetra-methylenglykol)polyetherpolyole (wie (HO-(CH₂-CH₂-CH₂-CH₂-O)ₓ-H) oder besteht daraus.

Geeignete Poly(tetramethylenglykol)polyetherpolyole sind beispielsweise durch Polymerisation von Tetrahydrofuran mittels kationischer Ringöffnung erhältlich sind.

Bevorzugt enthält die Komponente V2B) ein Gemisch aus Poly(tetramethylenglykol)-polyetherpolyolen oder besteht daraus, wobei sich die Poly(tetramethylenglykol)polyetherpolyole in ihren zahlenmittleren Molekulargewichten unterscheiden.

Weiterhin bevorzugt enthält die Komponente V2B) ein Gemisch aus Poly(tetramethylenglykol)polyetherpolyolen I mit einem zahlenmittleren Molekulargewichte Mₙ in einem Bereich von ≥ 400 und ≤ 1500 g/mol, besonders bevorzugt in einem Bereich von ≥ 600 und ≤ 1200 g/mol, ganz besonders bevorzugt in einem Bereich von 1000 g/mol und Poly(tetramethylenglykol)polyetherpolyole II mit einem zahlenmittleren Molekulargewichte Mₙ in einem Bereich von ≥ 1500 und ≤ 8000 g/mol, besonders bevorzugt in einem Bereich von ≥ 1800 und ≤ 3000 g/mol, ganz besonders bevorzugt von 2000 g/mol.

Das Gewichtsverhältnis der Poly(tetramethylenglykol)polyetherpolyole I zu den Poly(tetramethylen-glykol)polyetherpolyolen II liegt bevorzugt im Bereich von 0,1 bis 10, besonders bevorzugt im Bereich von 0,2 bis 10, ganz besonders bevorzugt im Bereich von 1 bis 6.

Erfindungsgemäß wird zur Herstellung des Polyurethanharnstoffs eine aminofunktionelle Kettenverlängerer-Komponente V2C) mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist, eingesetzt.

Bevorzugt weist die aminofunktionelle Kettenverlängerer-Komponente V2C) isocyanatreaktive Aminogruppen in einem Bereich von 2 bis 4, oder bevorzugt in einem Bereich von 2,05 bis 3, oder bevorzugt in einem Bereich von 2,1 bis 2,3, oder bevorzugt von 2 auf. Sind mehr als 2 isocyanatreaktive Aminogruppen in der aminofunktionelle Kettenverlängerer-Komponente V2C) enthalten so weist mindestens eine der mehr als 2 isocyanatreaktive Aminogruppen eine niedrigere Reaktivität gegenüber Isocyanatgruppen auf als die anderen beiden isocyanatreaktiven Aminogruppen auf. Dies führt insbesondere dazu, dass selbst bei einer Zahl von mehr als 2 isocyanatreaktiver Aminogruppen vor allem eine Kettenverlängerung stattfindet und weniger eine Vernetzung der zur Verfügung stehenden Iyocyanatgruppen.

Die aminofunktionellen Verbindungen der Komponente V2C) Komponente werden bevorzugt aus primären und/oder sekundären Diaminen ausgewählt. Insbesondere umfassen die aminofunktionellen Verbindungen V2C) mindestens ein Diamin.

Bevorzugt umfasst die aminofunktionelle Komponente V2C) wenigstens eine aminofunktionelle Verbindung C2), die ionische und/oder ionogene Gruppen aufweist.

Bevorzugt umfasst die aminofunktionelle Komponente V2C) sowohl aminofunktionelle Verbindungen C2), die ionische und/oder ionogene Gruppe aufweisen, als auch aminofunktionelle Verbindungen C1), die keine ionische oder ionogene Gruppe aufweisen.

Beispielsweise können als Komponente C1) organische Di- oder Polyamine wie beispielsweise 1,2-Ethylendiamin, 1,2- und 1,3-Diaminopropan, 1,4-Diaminobutan, 1,6-Diaminohexan, Isophorondiamin (IPDA), Isomerengemisch von 2,2,4- und 2,4,4-Trimethylhexamethylendiamin, 2-Methylpentamethylendiamin, Diethylentriamin, 4,4-Diaminodicyclohexylmethan und/oder Dimethylethylendiamin oder Mischungen aus mindestens zwei hiervon eingesetzt werden.

Bevorzugt ist die Komponente C1) ausgewählt aus der Gruppe bestehend aus 1,2-Ethylendiamin, Bis(4-aminocyclohexyl)methan, 1,4-Diaminobutan, IPDA, Ethanolamin, Diethanolamin und Diethylentriamin oder einer Mischung aus mindestens zwei hiervon.

Weiterhin bevorzugt enthält die Komponente C1) ≥ 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin oder IPDA oder ein Gemisch aus 1,2-Ethylendiamin und IPDA, wobei die Summe der beiden Amine in Bezug auf die Gesamtmenge an C1) bevorzugt in den genannten Bereichen vorliegt. Bevorzugt enthält die Komponente C1) ≥ 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin.

Bevorzugt umfasst die hydrophilierende Komponente C2) mindestens eine anionisch hydrophilierende Verbindung. Weiterhin bevorzugt beinhaltet die hydrophilierende Komponente C2) eine anionisch hydrophilierende Verbindung zu mindestens 80 Gew.-%, oder bevorzugt zu mindestens 90 Gew.-%, bezogen auf das Gesamtgewicht der Komponente C2). Besonders bevorzugt besteht die Komponente C2) aus ausschließlich anionisch hydrophilierenden Verbindungen.

Geeignete anionisch hydrophilierende Verbindungen enthalten wenigstens eine anionische oder ionogene Gruppe, die in eine anionische Gruppe überführt werden kann. Des Weiteren bevorzugt weisen geeignete anionisch hydrophilierende Verbindungen wenigstens zwei Aminogruppen und besonders bevorzugt zwei Aminogruppen auf. Besonders bevorzugt umfasst die hydrophilierende Komponente C2) eine anionisch hydrophilierende Verbindung, die wenigstens eine anionische oder ionogene Gruppe und wenigstens zwei Aminogruppen aufweist oder besteht daraus.

Geeignete anionisch hydrophilierende Verbindungen als Komponente C2), im Weiteren auch Hydrophilierungsmittel C2) genannt, enthalten bevorzugt eine Sulfonsäure- oder Sulfonatgruppe, besonders bevorzugt eine Natriumsulfonatgruppe. Geeignete anionisch hydrophilierende Verbindungen als Komponente C2) sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure oder 1,2- oder 1,3-Propylendiamin-β-ethylsulfonsäure oder Mischungen aus mindestens zwei hiervon.

Besonders bevorzugte anionische Hydrophilierungsmittel C2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure. Ganz besonders bevorzugt wird 2-(2-Aminoethylamino)ethylsulfonsäure oder deren Salze als anionisches Hydrophilierungsmittel C2) eingesetzt.

Gegebenenfalls kann die anionische Gruppe in der Komponente C2) auch eine Carboxylat- bzw. Carbonsäuregruppe sein. Die Komponente C2) wird dann bevorzugt aus Diaminocarbonsäuren ausgewählt. Bei dieser alternativen Ausführungsform müssen jedoch die Carbonsäure-basierenden Komponenten C2) in höheren Konzentrationen eingesetzt werden, verglichen mit solchen Komponenten C2), die Sulfonat- oder Sulfonsäuregruppen tragen. Besonders bevorzugt werden daher zur Herstellung des Polyurethanharnstoffs keine hydrophilierenden Verbindungen, die ausschließlich Carboxylatgruppen als anionische Gruppen der Komponente C2) tragen eingesetzt.

Bevorzugt wird zur Herstellung des erfindungsgemäß verwendeten Polyurethanharnstoffs in einem Bereich von ≥ 0,1 und ≤ 10 Gew.-% der Komponente C2) und besonders bevorzugt in einem Bereich von ≥ 0,5 und ≤ 4 Gew.-% der Komponente C2), jeweils bezogen auf die Gesamtmasse des Polyurethanharnstoffs, eingesetzt.

Zur Hydrophilierung können auch Mischungen aus anionischen Hydrophilierungsmitteln C2) und weiteren Hydrophilierungsmitteln V2D), welche von C2) verschieden sind, verwendet werden.

Geeignete weitere Hydrophilierungsmittel V2D) sind beispielsweise nichtionische hydrophilierende Verbindungen D1) und/oder hydroxyfunktionelle ionische oder ionogene Hydrophilierungsmittel D2). Bevorzugt handelt es sich bei der Komponente V2D) um nichtionisch hydrophilierende Komponenten D1).

Geeignete hydroxyfunktionelle ionische oder ionogene Hydrophilierungsmittel als Komponente D2) sind beispielsweise Hydroxycarbonsäuren wie Mono- und Dihydroxycarbonsäuren, wie 2-Hydroxyessigsäure, 3-Hydroxypropansäure, 12-Hydroxy-9-octadecansäure (Rizinolsäure), Hydroxypivalinsäure, Milchsäure, Dimethylolbuttersäure und/oder Dimethylolpropionsäure oder Gemische von mindestens zwei hieraus. Bevorzugt sind Hydroxypivalinsäure, Milchsäure und/oder Dimethylolpropionsäure, besonders bevorzugt ist Dimethylolpropionsäure. Bevorzugt werden keine hydroxyfunktionellen ionische oder ionogenen Hydrophilierungsmittel D2), insbesondere bevorzugt keine Hydrophilierungsmittel, die Carboxylat und Hydroxygruppen aufweisen, wie beispielsweise Dimethylolpropionsäure eingesetzt. Bevorzugt ist die Menge an hydroxyfunktionellen ionischen oder ionogenen Hydrophilierungsmittel D2) in einem Bereich von 0 bis 1 Gew.-%, oder bevorzugt in einem Bereich von 0 bis 0,5 Gew.-%, bezogen auf die Gesamtmasse des Polyurethanharnstoffs in dem Polyurethanharnstoff enthalten.

Geeignete nichtionisch hydrophilierende Verbindungen als Komponente V2D) sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether, Methanol oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

In einer bevorzugten Ausführungsform der Erfindung enthält der erfindungsgemäß verwendete Polyurethanharnstoff in einem Bereich von ≥ 0 und ≤ 20 Gew.-% der Komponente V2D), bevorzugt in einem Bereich von ≥ 0 und ≤ 10 Gew.-% der Komponente V2D) und ganz besonders bevorzugt in einem Bereich von ≥ 0 und ≤ 5 Gew.-% der Komponente V2D), jeweils bezogen auf die Gesamtmasse des Polyurethanharnstoffs. In einer weiteren bevorzugten Ausführungsform wird Komponente V2D) zur Herstellung des Polyurethanharnstoffs nicht verwendet.

Als Komponente V2E) können wahlweise Polyole, insbesondere nicht-polymere Polyole, des genannten Molekulargewichtsbereichs von 62 bis 399 g/mol mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Bevorzugt enthält der erfindungsgemäß verwendete Polyurethanharnstoff ≤ 10 Gew.-% der Komponente V2E), bevorzugt ≤ 5 Gew.-%, besonders bevorzugt 0 Gew.-% der Komponente V2E), jeweils bezogen auf die Gesamtmasse des Polyurethanharnstoffs. Bevorzugt beinhaltet der Polyurethanharnstoff die Komponente V2E) in einem Bereich von 0,1 bis 10 Gew.-%, bevorzugt in einem Bereich von 0,2 bis 8 Gew.-%, bevorzugt in einem Bereich von 0,1 bis 5 Gew.-%, bezogen jeweils auf die Gesamtmasse des Polyurethanharnstoffs. In einer weiteren bevorzugten Ausführungsform wird Komponente V2E) zur Herstellung des Polyurethanharnstoffs nicht verwendet.

Bevorzugt wird zur Herstellung des erfindungsgemäß verwendeten Polyurethanharnstoffs in einem Bereich von ≥ 0,5 und ≤ 20 Gew.-% der Summe der Komponenten C1) und gegebenenfalls V2E) und besonders bevorzugt in einem Bereich von ≥ 1 und ≤ 15 Gew.-% der Summe der Komponenten C1) und gegebenenfalls V2E), jeweils bezogen auf die Gesamtmasse des Polyurethanharnstoffs, eingesetzt.

Als Komponente V2F) können weitere polymere Polyole, welche unterschiedlich sind von V2B) eingesetzt werden.

Beispiele sind polymere Polyole, die nicht unter die Definition von V2B) und bevorzugt auch nicht unter die Definition von V2E) fallen, weil sie keine Polyetherpolyole sind - beispielsweise die in der Polyurethanlacktechnologie an sich bekannten Polyesterpolyole, Polyacrylatpolyole, Polyurethanpolyole, Polycarbonatpolyole, Polyesterpolyacrylatpolyole, Polyurethanpolyacrylatpolyole, Polyurethanpolyesterpolyole, Polyurethanpolycarbonatpolyole und Polyesterpolycarbonatpolyole.

Bevorzugt handelt es sich bei der Komponente V2F) nicht um polymere Polyole die Estergruppen aufweisen, insbesondere nicht um Polyesterpolyole.

Die Komponenten V2B) und V2F) enthalten erfindungsgemäß zusammen ≤ 30 Gew.-%, bevorzugt ≤ 10 Gew.-%, und besonders bevorzugt ≤ 5 Gew.-%, an Komponente V2F), bezogen auf die Gesamtmasse der Komponenten V2B) und V2F). Ganz besonders bevorzugt wird die Komponente V2F) zur Herstellung des Polyurethanharnstoffs nicht eingesetzt.

Bevorzugt wird zur Herstellung des erfindungsgemäß verwendeten Polyurethanharnstoffs in einem Bereich von ≥ 55 und ≤ 90 Gew.-% der Summe der Komponenten V2B) und gegebenenfalls V2F) und besonders bevorzugt in einem Bereich von ≥ 60 und ≤ 85 Gew.-% der Summe der Komponenten V2B) und gegebenenfalls V2F), jeweils bezogen auf die Gesamtmasse des Polyurethanharnstoffs, eingesetzt.

Bei der Komponente V2G) handelt es sich um Verbindungen, die genau eine isocyanatreaktive Gruppe aufweisen, oder um Verbindungen, die mehr als eine isocyanatreaktive Gruppe aufweisen, wobei nur eine der isocyanatreaktiven Gruppen unter den gewählten Umsetzungsbedingungen mit den in der Reaktionsmischung vorhandenen Isocyanatgruppen reagiert.

Bei den isocyanatreaktiven Gruppen der Komponente V2G) kann es sich dabei um jede funktionelle Gruppe handeln, die mit einer Isocyanatgruppe reagieren kann, wie beispielsweise Hydroxygruppen, Thiolgruppen oder primäre und sekundäre Aminogruppen.

Isocyanatreaktive Gruppen im Sinne der Erfindung sind dabei insbesondere bevorzugt primäre oder sekundäre Aminogruppen, die mit Isocyanatgruppen unter Bildung von Harnstoffgruppen reagieren. Neben der Aminogruppe können die Verbindungen der Komponente V2G) auch andere prinzipiell isocyanatreaktive Gruppen, wie OH-Gruppen aufweisen, wobei nur eine der isocyanatreaktiven Gruppen unter den gewählten Umsetzungsbedingungen mit den in der Reaktionsmischung vorhandenen Isocyanatgruppen reagiert. Dies kann beispielsweise durch Reaktion entsprechender Aminoalkohole bei relativ niedrigen Temperaturen erfolgen, beispielsweise bei 0 bis 60°C, bevorzugt bei 20 bis 40°C. Bevorzugt wird dabei in Abwesenheit von Katalysatoren gearbeitet, welche die Reaktion von Isocyanatgruppen mit Alkoholgruppen katalysieren würden.

Beispiele für geeignete Verbindungen der Komponente V2G) sind primäre/sekundäre Amine, wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Diethanolamin, 3-Amino-1-methylaminopropan, 3-Amino-1-ethylaminopropan, 3-Amino-1-cyclohexylaminopropan, 3-Amino-1- methylaminobutan, Ethanolamin, 3-Aminopropanol oder Neopentanolamin oder Mischungen aus mindestens zwei hiervon.

Geeignete monofunktionellen Verbindungen sind auch Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol sowie Mischungen aus mindestens zwei hiervon.

Bevorzugt wird zur Herstellung des erfindungsgemäß verwendeten Polyurethanharnstoffs ≥ 0,1 und ≤ 20 Gew.-% der Komponente V2G) und besonders bevorzugt ≥ 0,3 und ≤ 10 Gew.-% der Komponente V2G), jeweils bezogen auf die Gesamtmasse des Polyurethanharnstoffs, eingesetzt.

Bevorzugt wird die Komponente V2H) eingesetzt. Bevorzugt beträgt das molare Verhältnis von Komponente V2G) zu Komponente V2H) 5:1 bis 1:5, besonders bevorzugt 1,5:1 bis 1:4 und ganz besonders bevorzugt 1:1 bis 1:3.

Bevorzugt werden zur Herstellung der erfindungsgemäß verwendeten Polyurethanharnstoffe die Komponenten V2A) bis V2H) in den folgenden Mengen eingesetzt, wobei sich die Einzelmengen stets zu 100 Gew.-% addieren:
5 bis 40 Gew.-% Komponente V2A),
55 bis 90 Gew.-% Summe der Komponenten V2B) und gegebenenfalls V2F),
0,5 bis 20 Gew.-% Summe der Komponenten C1) und gegebenenfalls V2E),
0,1 bis 10 Gew.-% Komponente C2),
0 bis 20 Gew.-% Komponente V2D),
0,1 bis 20 Gew.-% der Komponente V2G) und
0 bis 10 Gew.-% Komponente V2H).

Ganz besonders bevorzugt ist der erfindungsgemäß verwendete Polyurethanharnstoff erhältlich durch Umsetzung ausschließlich der Komponenten A) bis H). Es werden dann keine weiteren Komponenten zur Herstellung des Polyurethanharnstoffs eingesetzt.

Das zahlenmittlere Molekulargewicht der erfindungsgemäß bevorzugt verwendeten Polyurethanharnstoffe beträgt bevorzugt von ≥ 2000 bis ≤ 300000 g/mol, bevorzugt von ≥ 5000 bis ≤ 150000 g/mol.

Der erfindungsgemäß verwendete Polyurethanharnstoff ist bevorzugt amorph und weist einen Tg ≤-25 °C, besonders bevorzugt von ≤ - 50 °C und ganz besonders bevorzugt von ≤ - 70 °C auf.

Amorph bedeutet im Sinne dieser Erfindung, dass der Polyurethanharnstoff im in der im folgenden ausgeführten Messmethode genannten Temperaturbereich keine oder nur so geringe kristalline Anteile ausbildet, dass mittels der beschriebenen DSC Messungen nur ein oder mehrere Glasübergangspunkte T_{g}, aber keine Schmelzbereiche mit einer Schmelzenthalpie ≥ 20J/g in dem genannten Temperaturbereich gefunden werden können.

Die Glasübergangstemperatur T_{g} wird im Rahmen dieser Erfindung mittels dynamischer Differenzkalorimetrie in Anlehnung an die DIN EN 61006, Verfahren A, bestimmt, wobei ein DSC Gerät verwendet wird, das zur Bestimmung von T_{g} mit Indium und Blei kalibriert ist und wobei drei unmittelbar aufeinander folgende Durchläufe einer Aufheizungen von -100°C bis +150°C, bei einer Heizrate von 20 K/min, mit anschließender Abkühlung mit einer Kühlrate von 320 K/min vorgenommen und die dritte Aufheizkurve zur Bestimmung der Werte verwendet wird und wobei als T_{g} die Temperatur bei der halben Höhe einer Glasübergangsstufe bestimmt wird.

Sollte der Polyurethanharnstoff in Form einer Dispersion vorliegen, wird bei der Probenpräparation für die DSC Messungen besonders vorgegangen. Bei der Bestimmung der Glasübergangstemperatur T_{g} von Dispersionen mittels DSC kann die T_{g} des Polymers durch die kalorischen Effekte des Dispergens (Wasser, Neutralisationsmittel, Emulgatoren, Colöser, etc.) maskiert bzw. wegen der Mischbarkeit mit dem Polymer deutlich abgesenkt werden. Daher wird das Dispergens vor der DSC-Messung bevorzugt durch geeignete Trocknung zunächst vollständig entfern, denn auch geringe Restmengen Dispergens wirken als Weichmacher und können die Glastemperatur dadurch absenken. Die Dispersion wird daher bevorzugt mit 100 µm Nassschichtdicke (NSD) auf eine Glasplatte gerakelt, abgelüftet und dann für zwei Tage in einer Trockenbox bei RT und 0% relativer Luftfeuchtigkeit (rF) schonend getrocknet. Nach dieser Probenpräparation kann in der ersten Aufheizung der DSC Messung noch einen breiten endothermen Verdampfungsbereich von Restfeuchte im Film. Um die bestimmten Werte möglichst von solchen Einflüssen frei zu erhalten, wir daher die dritte Aufheizkurve ausgewertet.

Der erfindungsgemäß zur Herstellung des Produktes eingesetzte Polyurethanharnstoff liegt bevorzugt in einem physiologisch akzeptablen isocyanatreaktive-Gruppen enthaltenden Medium vor. Besonders bevorzugt handelt es sich bei dem Medium um Wasser und ganz besonders bevorzugt liegt der Polyurethanharnstoff als wässrige Dispersion vor. Wasser bildet im Allgemeinen, neben wahlweise vorhandenen anderen flüssigen Medien, wie beispielsweise Lösungsmitteln, den Hauptbestandteil (≥ 50 Gew.-%) des Mediums, bezogen auf die Gesamtmenge des flüssigen isocyanatreaktive-Gruppen enthaltenden Mediums, gegebenenfalls auch das alleinige flüssige isocyanatreaktive-Gruppen enthaltenden Medium.

Das erfindungsgemäße visko-elastische Element selbst enthält den Polyurethanharnstoff an sich, der keine oder nur noch Restmengen dieses Mediums enthält.

Bevorzugt ist der verwendete Polyurethanharnstoff daher in Wasser dispergierbar, was im Rahmen dieser Erfindung bedeutet, dass der Polyurethanharnstoff bei 23°C eine sedimentationsstabile Dispersion in Wasser, insbesondere entionisiertem Wasser, bilden kann.

Die erfindungsgemäß verwendeten Polyurethanharnstoffe sind bevorzugt erhältlich, indem isocyanatfunktionelle Polyurethanpräpolymere a) aus den Komponenten V2A), V2B) und gegebenenfalls V2D) und/oder C2), sowie gegebenenfalls den Verbindungen V2E) und/oder V2H) hergestellt werden (Schritt a) und deren freie NCO-Gruppen anschließend ganz oder teilweise mit der aminofunktionellen Kettenverlängerer-Komponente V2C), sowie der Komponente V2G) und gegebenenfalls der Komponente V2D) und V2H) umgesetzt werden (Schritt b)).

Wobei wenn die Komponente V2H) erst in Schritt b) eingesetzt wird, diese bevorzugt vor der Zugabe der Komponente V2C) zugegeben und mit dem Prepolymer a) umgesetzt wird.

In einer bevorzugten Ausführungsform der Erfindung erfolgt im Schritt b) die Umsetzung mit einem Diamin oder mehreren Diaminen (Komponente V2C) unter Kettenverlängerung wobei außerdem die monofunktionelle Komponente V2G) als Kettenabbrecher zur Molekulargewichtssteuerung zugesetzt wird.

Die Komponenten Die Komponenten V2A) bis V2H) sind dabei wie oben angegeben definiert. Es gelten auch die oben genannten bevorzugten Ausführungsformen.

Bevorzugt wird im Schritt b) der Umsetzung des Präpolymers a) zur Herstellung des Polyurethanharnstoffs eine Mischung aus Komponenten C1), C2) und V2G) zur Umsetzung gebracht. Durch die Verwendung der Komponente C1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten C1), C2) und V2G) lässt sich eine optimale Balance zwischen Hydrophilie und Kettenlänge einstellen.

Bevorzugt weist das erfindungsgemäß verwendete Polyurethanpräpolymere a) endständige Isocyanatgruppen auf, d.h. die Isocyanatgruppen liegen an den Kettenenden des Präpolymers. Besonders bevorzugt weisen sämtliche Kettenenden des Präpolymers Isocyanatgruppen auf.

V2A) bis V2H) sind dabei wie oben angegeben definiert. Es gelten auch die oben genannten bevorzugten Ausführungsformen.

Bevorzugt wird im Schritt b) der Umsetzung des Präpolymers a) zur Herstellung des Polyurethanharnstoffs eine Mischung aus Komponenten C1), C2) und V2G) zur Umsetzung gebracht. Durch die Verwendung der Komponente C1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten C1), C2) und V2G) lässt sich eine optimale Balance zwischen Hydrophilie und Kettenlänge einstellen.

Bevorzugt weist das erfindungsgemäß verwendete Polyurethanpräpolymere a) endständige Isocyanatgruppen auf, d.h. die Isocyanatgruppen liegen an den Kettenenden des Präpolymers. Besonders bevorzugt weisen sämtliche Kettenenden des Präpolymers Isocyanatgruppen auf.

Über die hydrophilierende Komponente C2) und/oder V2D) kann die Hydrophilie des Präpolymers gesteuert werden. Daneben spielen für die Hydrophilie des Präpolymers natürlich auch noch weitere Komponenten, speziell auch die Hydrophilie der Komponente V2B) eine Rolle.

Bevorzugt sind die isocyanatfunktionellen Polyurethanpräpolymere a) wasserunlöslich und nicht in Wasser dispergierbar.

Im Rahmen der Erfindung bedeutet der Begriff "wasserunlösliches, nicht wasserdispergierbares Polyurethanpräpolymer" insbesondere, dass die Wasserlöslichkeit des erfindungsgemäß verwendeten Präpolymers bei 23°C weniger als 10 g / Liter, bevorzugter weniger als 5 g / Liter beträgt und das Präpolymer bei 23° keine sedimentationsstabile Dispersion in Wasser, insbesondere entionisiertem Wasser, ergibt. Mit anderen Worten setzt sich das Präpolymer, beim Versuch es in Wasser zu dispergieren, ab. Die Wasserunlöslichkeit bzw. fehlende Dispergierbarkeit in Wasser bezieht sich auf entionisiertes Wasser ohne Zusatz von Tensiden.

Weiterhin weist das erfindungsgemäß verwendete Polyurethanpräpolymere a) bevorzugt im Wesentlichen weder ionische noch ionogene (zur Bildung von ionischen Gruppen befähigte) Gruppen auf. Im Rahmen der vorliegenden Erfindung bedeutet dies, dass der Anteil der ionischen und/oder ionogenen Gruppen, wie insbesondere anionischer Gruppen, wie Carboxylat oder Sulfonat, oder kationischer Gruppen weniger beträgt als 15 Milliequivalente pro 100 g Polyurethanpräpolymer a1), bevorzugt weniger als 5 Milliequivalente, besonders bevorzugt weniger als 1 Milliequivalent und ganz besonders bevorzugt weniger als 0,1 Milliequivalente pro 100 g Polyurethanpräpolymer a).

Im Falle saurer ionischer und/oder ionogener Gruppen beträgt die Säurezahl des Präpolymers zweckmäßig unter 30 mg KOH/g Präpolymer, bevorzugt unter 10 mg KOH/g Präpolymer. Die Säurezahl gibt die Masse Kaliumhydroxid in mg an, die zur Neutralisation von 1 g der zu untersuchenden Probe erforderlich ist (Messung nach DIN EN ISO 211). Die neutralisierten Säuren, also die entsprechenden Salze weisen naturgemäß keine oder eine reduzierte Säurezahl auf. Hier ist erfindungsgemäß die Säurezahl der korrespondierenden freien Säure entscheidend.

Dabei sind die wasserunlöslichen, nicht wasserdispergierbaren, isocyanatfunktionellen Polyurethanpräpolymeren a) bevorzugt ausschließlich erhältlich aus den Komponenten V2A), V2B) und gegebenenfalls V2D), V2E) und/oder V2H).

Die Komponenten sind dabei wie oben angegeben definiert. Es gelten auch die oben genannten bevorzugten Ausführungsformen.

Es werden folglich bevorzugt in dieser Ausführungsform keine ionisch hydrophilierenden Komponenten C2) oder auch D2) zur Herstellung des Präpolymers a) eingesetzt. Auch wird die Komponente V2G) in diesem Schritt nicht zugesetzt. Die Hydrophilierungsmittel D1) werden bevorzugt in solchen Mengen eingesetzt, dass das Präpolymer dennoch wasserunlöslich und nicht wasserdispergiebar ist. Besonders bevorzugt werden ≤ 10 Gew.-% der Komponente D1), ganz besonders bevorzugt ≤ 5 Gew.-% und weiterhin bevorzugt ≤ 2 Gew.-% der Komponente D1), jeweils bezogen auf die Gesamtmasse des Polyurethanharnstoffs, eingesetzt. Weiterhin bevorzugt wird die Komponente D1) zur Herstellung des Präpolymers a) nicht eingesetzt.

Für diese Ausführungsform der Erfindung weist die Komponente V2B) weder ionische noch ionogene Gruppen auf. Weiterhin werden bei dieser Ausführungsform der Erfindung als Komponente V2B) bevorzugt nur Polyetherpolyole, insbesondere Polyalkylenoxidether eingesetzt, die ≤ 10 mol-% und bezogen auf alle enthaltenen Alkylenoxideinheiten an Ethylenoxideinheiten und bevorzugt keine Ethylenoxideinheiten enthalten.

Die in dieser Ausführungsform der Erfindung bevorzugt verwendeten Polyurethanharnstoffe weisen folglich ionische oder ionogene Gruppen auf, bevorzugt anionische Gruppen auf, diese anionischen Gruppen werden dabei in die erfindungsgemäß verwendeten Polyurethanharnstoffe über die in Schritt b) eingesetzte hydrophilierende Komponente C2) eingeführt. Die erfindungsgemäß verwendeten Polyurethanharnstoffe weisen gegebenenfalls zusätzlich nicht-ionische Komponenten zu Hydrophilierung auf.

Besonders bevorzugt sind in den erfindungsgemäß verwendeten Polyurethanharnstoffen zur Hydrophilierung ausschließlich Sulfonatgruppen enthalten, welche in Schritt b) über entsprechende Diamine als Komponente C2) in den Polyurethanharnstoff eingeführt werden.

In einer alternativen, weniger bevorzugten Ausführungsform der Erfindung sind die zur Herstellung des erfindungsgemäßen Polyurethanharnstoffs eingesetzten Präpolymere a) wasserlöslich oder wasserdispergierbar. In dieser Ausführungsform werden die hydrophilierende Komponente V2D) und/oder C2) bei der Herstellung des Präpolymers a) in einer Menge eingesetzt, die ausreichend ist, damit das Präpolymer wasserlöslich oder wasserdispergierbar ist. Das Präpolymer a) weist dabei bevorzugt ionische oder ionogene Gruppen auf.

Geeignete hydrophilierende Komponenten V2D) und C2) sind für diese Ausführungsform der Erfindung die oben für V2D) und C2) genannten Verbindungen. Bevorzugt werden als hydrophilierende Komponenten wenigstens die oben unter D1) und/oder C2) genannten Verbindungen eingesetzt.

Die zur Herstellung der erfindungsgemäßen Produkte verwendeten Polyurethanharnstoffe werden bevorzugt vor, während oder nach Schritt b), besonders bevorzugt während oder nach Schritt b) in Wasser dispergiert. So wird eine Dispersion der Polyurethanharnstoffe erhalten.

Die Herstellung der Polyurethanharnstoff-Dispersionen kann dabei in einer oder mehreren Stufe/-n in homogener oder bei mehrstufiger Umsetzung, teilweise in disperser Phase durchgeführt werden. Nach Herstellung des Präpolymers a) erfolgt bevorzugt ein Dispergier-, Emulgier- oder Lösungsschritt. Im Anschluss erfolgt gegebenenfalls eine weitere Polyaddition oder Modifikation in disperser Phase. Dabei wird jeweils das für das entsprechende Präpolymer geeignete Löse- oder Dispergiermittel wie beispielsweise Wasser oder Aceton oder Mischungen daraus gewählt.

Dabei können alle aus dem Stand der Technik bekannten Verfahren wie z. B. Präpolymer-Mischverfahren, Acetonverfahren oder Schmelzdispergierverfahren verwendet werden. Bevorzugt wird das Aceton-Verfahren angewandt.

Für die Herstellung nach dem Aceton-Verfahren werden üblicherweise die Bestandteile V2B), gegebenenfalls V2D) und V2E) und die Polyisocyanatkomponente V2A) gegebenenfalls in Kombination mit der Komponente V2H) zur Herstellung eines isocyanatfunktionellen Polyurethanpräpolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend können die gegebenenfalls zu Beginn der Reaktion noch nicht zugegebenen Bestandteile von V2A), V2B) und gegebenenfalls V2H), V2D) und V2E) zu dosiert werden.

Bei der Herstellung des Polyurethan-Präpolymeren aus V2A), V2B) und gegebenenfalls V2H), V2D) und V2E) beträgt das Stoffmengenverhältnis von Isocyanat-Gruppen zu isocyanatreaktiven Gruppen bevorzugt 1,05 bis 3,5, besonders bevorzugt 1,1 bis 3,0 und ganz besonders bevorzugt 1,1 bis 2,5.

Die Umsetzung der Komponenten V2A), V2B) und gegebenenfalls V2H), V2D) und V2E) zum Präpolymer kann teilweise oder vollständig, bevorzugt aber vollständig erfolgen. Es können so Polyurethanpräpolymere, die freie Isocyanatgruppen enthalten, in Substanz oder in Lösung erhalten werden.

Sollten ionogene Gruppen, wie beispielsweise Carboxylgruppen, im Präpolymer vorliegen können diese in einem weiteren Schritt durch Neutralisation in ionische Gruppen überführt werden.

Im Neutralisationsschritt zur teilweisen oder vollständigen Überführung potentiell anionischer Gruppen in anionische Gruppen können Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt werden.

Als Neutralisationsmittel sind bevorzugt anorganische Basen, wie wässrige Ammoniaklösung oder Natrium- bzw. Kaliumhydroxid einsetzbar, besonders bevorzugt sind Natriumhydroxid und Kaliumhydroxid.

Die Stoffmenge der Basen beträgt bevorzugt 50 und 125 mol%, besonders bevorzugt zwischen 70 und 100 mol% der Stoffmenge der zu neutralisierenden Säuregruppen. Die Neutralisation kann, auch gleichzeitig mit der Dispergierung erfolgen, in dem das Dispergierwasser bereits das Neutralisationsmittel enthält.

Im Anschluss an die Neutralisation wird in einem weiteren Verfahrensschritt, falls noch nicht oder nur teilweise geschehen, das erhaltene Präpolymer mit Hilfe von aliphatischen Ketonen wie Aceton oder 2-Butanon gelöst.

Bei der Kettenverlängerung/-terminierung in Stufe b) werden die Komponenten V2C), V2G) und gegebenenfalls V2D) mit den noch verbliebenen Isocyanatgruppen des Präpolymers umgesetzt. Bevorzugt wird die Kettenverlängerung/-terminierung vor der Dispergierung in Wasser durchgeführt.

Geeignete Komponenten V2C) zur Kettenverlängerung sowie V2G) zum Kettenabbruch sind bereits oben aufgeführt. Es gelten analog auch die oben genannten bevorzugten Ausführungsformen.

Werden zur Kettenverlängerung anionische Hydrophilierungsmittel entsprechend der Definition C2) mit NH₂- oder NH-Gruppen eingesetzt, erfolgt die Kettenverlängerung der Präpolymere in Schritt b) bevorzugt vor der Dispergierung in Wasser.

Das Äquivalentverhältnis von NCO-reaktiven Gruppen der zur Kettenverlängerung und Kettenterminierung eingesetzten Verbindungen zu freien NCO-Gruppen des Präpolymers, liegt im allgemeinen zwischen 40 und 150 %, bevorzugt zwischen 50 und 110 %, besonders bevorzugt zwischen 60 und 100 %.

Die Komponenten C1), C2) und V2G), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel in Schritt b) mit verwendet werden, so beträgt der jeweilige Verdünnungsmittelgehalt in den eingesetzten Komponenten C1), C2) und V2G) bevorzugt 40 bis 95 Gew.-%.

Die Dispergierung erfolgt bevorzugt im Anschluss an die Kettenverlängerung und Kettenterminierung. Dazu wird das gelöste (beispielsweise in Aceton) und mit dem Aminen umgesetzte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Die erhaltenen wässrigen Polyurethanharnstoffdispersionen weisen bevorzugt einen Gehalt an flüchtigen organischen Verbindungen (VOC), wie beispielsweise flüchtige organische Lösemitteln, von weniger als 10 Gew.-%, bevorzugter von weniger als 3 Gew.-%, noch bevorzugter von weniger als 1 Gew.-% bezogen auf die auf wässrige Polyurethanharnstoffdispersion auf. VOCs im Sinne dieser Erfindung sind insbesondere organische Verbindung mit einem Anfangssiedepunkt von höchstens 250 °C bei einem Standarddruck von 101,3 kPa.

Die Bestimmung des Gehalts an flüchtigen organischen Verbindungen (VOC) erfolgt im Rahmen der vorliegenden Erfindung insbesondere durch gaschromatographische Analyse.

Der Polyurethanharnstoff wird zur Herstellung des Produkts bevorzugt als wässrige Dispersion eingesetzt.

Der pH-Wert der erfindungsgemäß verwendeten wässrigen Polyurethan-Dispersionen beträgt typischerweise weniger als 9,0, bevorzugt weniger als 8,5 und liegt besonders bevorzugt zwischen 5,5 und 8,0.

Um eine gute Sedimentationsstabilität zu erreichen, liegt die zahlenmittlere Teilchengröße der speziellen Polyurethanharnstoff-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malvern Inst. Limited).

Der Feststoffgehalt der Polyurethanharnstoff-Dispersionen beträgt bevorzugt 10 bis 70 Gew.-%, besonders bevorzugt 20 bis 60 Gew.-%und ganz besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethanharnstoff-Dispersionen weniger als 5 Gew.-%, besonders bevorzugt weniger als 0,2 Gew.-%, bezogen auf die Masse der Dispersionen, an ungebundenen organischen Aminen auf.

Die zur Herstellung der erfindungsgemäßen Produkte eingesetzte Polyurethanharnstoff-Dispersionen weist bei 23°C bei einer konstanten Scherrate von 10 s⁻¹ bevorzugt eine Viskosität von ≥ 1 und ≤ 10000 mPa s, besonders bevorzugt von ≥ 10 und ≤ 5000 mPa s und ganz besonders bevorzugt von ≥ 100 und ≤ 4000 mPa s auf. Die Viskosität wird dabei wie im Methodenteil beschrieben bestimmt.

In einer bevorzugten Ausführungsform des visko-elastische Elements liegen die Komponenten (V1) mit (V2) bei der Reaktion der Komponenten (V1) und (V2) in einem Gewichtsverhältnis von 5:1 bis 1:1, oder bevorzugt von 4:1 bis 1:1, oder bevorzugt von 3:1 bis 1:1, bezogen auf das Gesamtgewicht der beiden Komponenten (V1) und (V2).

In einer bevorzugten Ausführungsform des visko-elastische Elements weist das visko-elastische Element mindestens eine der folgenden Eigenschaften auf:
i. eine Dämpfung (aus Stauchhärte-Messung) von 0,40 bis 0,90, oder bevorzugt von 0,45 bis 0,80, oder bevorzugt von 0,45 bis 0,70, gemessen nach DIN EN ISO 3386-1:2015-10;
ii. eine Bruchspannung in einem Bereich von 0,12 MPa bis 0,40 MPa, oder bevorzugt von 0,13 bis 0,35 MPa, oder bevorzugt von 0,14 bis 0,30 MPa, oder bevorzugt von 0,15 bis 0,25 MPa gemessen nach DIN EN ISO 527-2;
iii. eine Bruchdehnung in einem Bereich von 150 % bis 300%, oder bevorzugt von 180 % bis 290 %, oder bevorzugt von 200 % bis 280 %, oder bevorzugt von 220 % bis 270 %, gemessen nach DIN EN ISO 527-2;
iv. eine Absorption von Wasser von ≤ 1800 % (g/g), oder bevorzugt von ≤ 1700 % (g/g), oder bevorzugt von ≤ 1500 % (g/g), bezogen auf Gewicht des trockenen Schaums gemessen nach DIN EN 13726-1;
v. eine Retention von ≤ 80 %, oder bevorzugt von ≤ 70 %, oder bevorzugt von ≤ 60 %, oder bevorzugt in einem Bereich von ≥ 20 % bis ≤ 80%, oder bevorzugt in einem Bereich von ≥ 30 % bis ≤ 70 %, bezogen auf die maximale Absorption aufweist, wie unter Methoden später beschrieben;
vi. eine Rückstellzeit nach Kompression des Ursprungvolumens um 50 % (bei einem Druck von 7 kPa) auf mindestens 90 % des Ursprungsvolumens bei Normaldruck und RT in einem Bereich von 2 bis 50 Sekunden, oder bevorzugt von 3 bis 40, oder bevorzugt von 4 bis 30;
vii. ein Volumen in einem Bereich von 0,1 cm³ bis 1 m³, oder bevorzugt von 0,15 cm³ bis 0,5 m³, oder bevorzugt von 0,2 cm³ bis 0,1 m³;
viii. eine Dichte in einem Bereich von 150 bis 400 g/l, oder bevorzugt von 170 bis 380 g/l, oder bevorzugt von 180 bis 350 g/l, oder bevorzugt von 200 bis 300 g/l;
ix. einen Verlustfaktor ≥ 0,140 (aus rheologischen Untersuchungen), bevorzugt einen Verlustfaktor in einem Bereich von 0,140 bis 0,400, oder bevorzugt von 0,150 bis 0,350, oder bevorzugt von 0,160 bis 0,300, gemessen nach DIN EN ISO 6721-1 aufweist;
x. einen E-Modul nach DIN EN ISO 527-2 in einem Bereich von 0,08 MPa bis 0,25 MPa, oder bevorzugt von 0,08 MPa bis 0,2 MPa, oder bevorzugt von 0,08 MPa bis 0,15 MPa.

Das visko-elastische Element kann sämtliche möglichen Kombinationen der Merkmale i. bis x. aufweisen. Bevorzugt weist das visko-elastische Element mindestens das Merkmal vi., weiterhin bevorzugt die Merkmalskombination vi. und mindestens eines der Merkmale i., ii. oder iii. auf. Bevorzugt weist das visko-elastische Element die Merkmalskombination i. bis iii. und vi. auf. Weiterhin bevorzugt weist das visko-elastische Element die Merkmalskombination i. bis vi. auf. Bevorzugt weist das visko-elastische Element sämtliche der Merkmale i. bis x. auf.

In einer bevorzugten Ausführungsform des visko-elastische Elements enthalten die Komponenten V2B) und V2F) zusammen ≤ 30 Gew.-% an Komponente V2F), bezogen auf die Gesamtmasse der Komponenten V2B) und V2F) enthalten.

In einer bevorzugten Ausführungsform des visko-elastische Elements ist die Komponente V2A) Isophorondiisocyanat und/oder Hexamethylendiisocyanat. Bevorzugt weist die Komponente V2A) ausschließlich Isophorondiisocyanat und/oder Hexamethylendiisocyanat auf.

In einer bevorzugten Ausführungsform des visko-elastische Elements enthält die Komponente V2B) Poly(tetramethylenglykol)polyetherpolyole (wie (HO-(CH₂-CH₂-CH₂-CH₂-O)ₓ-H) oder besteht daraus. Bevorzugt enthält die Komponente V2B) das Poly(tetramethylenglykol)polyetherpolyole zu mindestens 50 Gew.-%, oder bevorzugt zu mindestens 70 Gew.-%, oder bevorzugt zu mindestens 90 Gew.-%, bezogen auf die Gesamtmasse der Komponente V2B).

In einer bevorzugten Ausführungsform des visko-elastische Elements ist die Komponente (V1) ein isocyanatfunktionelles Prepolymer mit einem Gewichtsanteil an niedermolekularen, aliphatischen Diisocyanaten mit einer Molmasse von ≥ 140 bis ≤ 278 g/mol, bevorzugt ≥ 168 bis ≤ 258 g/mol von ≤ 1.0 Gew.-%, bezogen auf das Prepolymer ist, erhältlich durch Umsetzung von
- V1A): niedermolekularen oder aliphatischen Diisocyanaten einer Molmasse von ≥ 140 bis ≤ 278 g/mol oder bevorzugt ≥ 168 bis ≤ 258 g/mol mit
- V1B): di- bis hexafunktionellen Polyalkylenoxiden einer OH-Zahl von ≥ 22.5 bis ≤ 112 mg KOH/g und einem Ethylenoxidanteil von ≥ 50 bis ≤ 100 mol%, bezogen auf die Gesamtmenge der enthaltenen Oxyalkylengruppen,
- V1C): gegebenenfalls heterocyclische, 4-Ring oder 6-Ring-Oligomere von niedermolekularen oder aliphatischen Diisocyanaten mit einer Molmasse von ≥ 140 bis ≤ 278 g/mol, oder bevorzugt ≥ 168 bis ≤ 258 g/mol,
- V1D): gegebenenfalls Katalysatoren,
- V1E): gegebenenfalls Alkalimetallsalze schwacher anorganischer Säuren,

- V1F): gegebenenfalls Tenside,
- V1G): gegebenenfalls ein- oder mehrwertige Alkohole,
- V1H): die folgenden Komponenten:
V1H1) ein oder mehrere niedermolekulare, aliphatische Diisocyanate einer Molmasse von ≥ 140 bis ≤ 278 g/mol, bevorzugt ≥ 168 bis ≤ 258 g/mol und/oder daraus herstellbare Polyisocyanate mit einer Isocyanatfunktionalität von ≥ 2 bis ≤ 6; und/oder
V1H2) ein oder mehrere monofunktionelle Polyalkylenoxide mit einer OH-Zahl von ≥ 10 bis ≤ 250 und einem Ethylenoxidanteil von ≥ 50 bis ≤ 100 mol%, bezogen auf die Gesamtmenge der enthaltenen Oxyalkylengruppen; und/oder
V1H3) eine hydrophile Isocyanatkomponente, welche durch die Umsetzung von unter V1H1) genannten Komponenten mit unter V1H2) genannten Komponenten erhältlich ist.

Die Herstellung der unter V1H) genannten hydrophilen Polyisocyanate erfolgt typischerweise durch Umsetzung von 1 Mol OH-Gruppen der monofunktionellen Polyalkylenoxidkomponente V1H2) mit 1.25 bis 15 Mol, bevorzugt mit 2 bis 10 Mol und besonders bevorzugt 2 bis 6 Mol NCO-Gruppen eines Polyisocyanates V1H1) mit einer Isocyanatfunktionalität von 2 bis 6, basierend auf aliphatischen Diisocyanaten. Beispielhaft für derartige Polyisocyanate V1H1) sind Biuret-Strukturen, Isocyanurate bzw. Uretdione basierend auf aliphatischen Diisocyanaten. Dabei werden das Polyisocyanat V1H1) und das Polyalkylenoxid V1H2) bevorzugt über eine Urethangruppe bzw. eine Harnstoffgruppe miteinander verknüpft, wobei besonders die Verknüpfung über Urethangruppen bevorzugt ist.

Der NCO-Gehalt der isocyanatfunktionellen Prepolymere V1A) beträgt bevorzugt 1.5 bis 4.5 Gew.-%, besonders bevorzugt 1.5 bis 3.5 Gew.-% und ganz besonders bevorzugt 1.5 bis 3.0 Gew.-%.

Beispiele für niedermolekulare, aliphatische Diisocyanate der Komponente V1A) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Zur Beschleunigung der Harnstoff - bzw. Urethanbildung können in Komponente V1D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, Aminen, Amidinen und Guanidinen. Beispielhaft zu nennen sind Zinndibutyldilaurat (DBTL), Zinnacetat, 1 ,8-Diazabi-cyclo[5.4.0]undecen-7 (DBU), 1,5-Diazabicyclo[4.3.0]nonen-5 (DBN), 1 ,4-Diazabicy-clo[3.3.0]octen-4 (DBO), N-Ethylmorpholin (NEM), Triethylendiamin (DABCO), Pentamethylguanidin (PMG), Tetramethylguanidin (TMG), Cyclotetramethylguanidin (TMGC), n-Decyl-tetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Bevorzugt ist der Einsatz von Aminen, Amidinen, Guanidinen oder deren Mischungen als Katalysatoren der Komponente V1D). Bevorzugt ist auch die Verwendung von 1,8-Diazabicyclo[5.4.0]undecen-7 (DBU).

Es kann aber auch auf Katalysatoren verzichtet werden; dies ist bevorzugt.

Als Komponente V1E) werden Alkalimetallsalze schwacher anorganischer Säuren eingesetzt. Hierunter werden Alkalimetallsalze von anorganischen Säuren verstanden, deren korrespondierende freie Säuren in Wasser bei 25 °C einen pKs-Wert von > 4.0 aufweisen. Beispiele besonders geeigneter Alkalimetallsalze schwacher anorganischer Säuren sind Kaliumhydroxid, Natriumhydroxid, Kaliumcarbonat, Natriumcarbonat und Natriumhydrogencarbonat, wobei auch beliebige Mischungen dieser Salze mit eingeschlossen sind.

Zur Verbesserung der Schaumbildung, Schaumstabilität oder der Eigenschaften des resultierenden Polyurethan-Schaums können Verbindungen der Komponente V1F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere eingesetzt. Bevorzugt werden allein die EO/PO-Blockcopolymere als Komponente V1F) eingesetzt.

Zudem können zur Verbesserung der Schaumeigenschaften des resultierenden Polyurethan-Schaums Verbindungen der Komponente V1G) verwendet werden. Hierbei handelt es sich um grundsätzlich alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Bei der Herstellung der unter V1H) aufgeführten hydrophilen Polyisocyanate wird das Verhältnis der monofunktionellen Polyalkylenoxide V1H2) zu den niedermolekularen, aliphatischen Diisocyanaten V1H1) bevorzugt so eingestellt, dass auf 1 Mol OH-Gruppen der monofunktionellen Polyalkylenoxide 1.25 bis 15 Mol, bevorzugt 2 bis 10 Mol und besonders bevorzugt 2 bis 6 Mol NCO-Gruppen des niedermolekularen, aliphatischen Diisocyanats V1H1) kommen. Anschließend erfolgt die Allophanatisierung bzw. Biurethisierung und/oder Isocyanuratbildung bzw. Uretdionbildung. Werden die Polyalkylenoxide V1H2) über Urethangruppen an die aliphatischen Diisocyanate V1H1) gebunden, findet bevorzugt im Anschluss eine Allophanatisierung statt. Weiterhin ist bevorzugt, dass Isocyanurat-Struktureinheiten gebildet werden.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung erfolgt typischerweise bei 25 bis 140 °C, bevorzugt 60 bis 100 °C.

Beispiele für niedermolekulare, aliphatische Diisocyanate der Komponente V1H1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat. Beispiele für höhermolekulare Polyisocyanate V1H2) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente V1H1) höhermolekulare Verbindungen mit Biuret-, Iminooxadiazindion-, Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxide V1H2) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden V1H2) durch Alkoxylierung geeigneter Startermoleküle ist literaturbekannt (z.B. Ullmanns Enzyklopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet. Die monofunktionellen Polyalkylenoxide V1H2) besitzen typischerweise zahlenmittlere Molekulargewichte von 220 bis 3700 g/mol, bevorzugt von 500 bis 2800 g/mol.

Die monofunktionellen Polyalkylenoxide V1H2) besitzen bevorzugt eine OH-Gruppe als isocyanatreaktive Gruppe.

Ein weiterer Gegenstand der Erfindung betrifft ein Verfahren zur Herstellung eines visko-elastischen Elements umfassend die Schritte:
I) Mischen eines isocyanatfunktionellen Polyurethan-Prepolymers (V1) mit mindestens einer Polyurethanharnstoff-Dispersion (V2) in Anwesenheit eines isocyanatreaktive-Gruppen enthaltenden Mediums unter Erhalt einer Polyurethan-Prepolymer-Medium-Mischung; wobei das Medium bevorzugt Wasser ist,
II) Aufbringen der Polyurethan-Prepolymer-Medium-Mischung aus Schritt I) auf ein Substrat;
III) Aushärtung der Polyurethan-Prepolymer-Medium-Mischung, wobei das Medium bevorzugt Wasser ist und die Polyurethan-Prepolymer-Medium-Mischung insbesondere eine Polyurethan-Prepolymer-Wasser-Mischung ist, aus Schritt II) unter Erhalt des visko-elastischen Elements;
IV) ggf. Entfernen des visko-elastischen Elements von dem Substrat.

In einer bevorzugten Ausführungsform des Verfahren erfolgt das Mischen in Schritt I) innerhalb von 3 bis 90, bevorzugt innerhalb von 5 bis 60, oder bevorzugt innerhalb von 6 bis 30 Sekunden, bevor die Mischung in Schritt II) auf das Substrat aufgebracht wird.

Das Mischen in Schritt I) kann mittels jeden Mischverfahrens erfolgen das der Fachmann hierfür auswählen würde. Bevorzugt erfolgt das Mischen mittels eines Scheibenrührers, Speedmixers, Ankerrührers, KPG-Rührers, eines statischen, dynamischen oder statisch-dynamischen Rührers oder eines Niederdruck-Zweikomponentenmischgeräts. Das Mischen in Schritt I) findet bevorzugt bei einer Temperatur in einem Bereich von 10 bis 100 °C, oder bevorzugt in einem Bereich von 20 bis 60 °C, oder bevorzugt in einem Bereich von 23 bis 50 °C statt. Das Mischen in Schritt I) findet bevorzugt in einem Druckbereich von Normaldruck bis 20 bar Druck statt. Bevorzugt findet das Mischen bei Normaldruck statt.

Das Aufbringen der Polyurethan-Prepolymer-Medium-Mischung aus Schritt I) auf ein Substrat in Schritt II) kann auf jede Art erfolgen, die der Fachmann hierfür auswählen würde. Bevorzugt ist das Aufbringen ausgewählt aus der Gruppe bestehend aus einem Gießen, einem Sprühen, einem Drucken oder einer Kombination aus mindestens zwei hiervon. Dass Gießen kann aus jedem Gefäß erfolgen, das der Fachmann hierfür einsetzen würde. Das Sprühen kann mittels jedes Sprühwerkzeugs erfolgen, das der Fachmann hierfür einsetzen würde. Bevorzugt findet das Sprühen mittels einer Düse mit einem Durchmesser in einem Bereich von 0,1 mm bis 10m, oder bevorzugt in einem Bereich von 0,5 mm bis 1 m, oder bevorzugt in einem Bereich von 1 mm bis 50 cm. Das Substrat kann jedes Substrat sein, das der Fachmann hierfür auswählen würde. Bevorzugt ist das Substrat aus einem Material, das keine Reaktion mit der Polyurethan-Prepolymer-Medium-Mischung, insbesondere der Polyurethan-Prepolymer-Wasser-Mischung, aus Schritt II) eingeht. Bevorzugt ist das Substrat so ausgewählt, dass es sich leicht von dem ausgehärteten visko-elastischen Element lösen lässt, ohne, dass das Element Schaden nimmt oder Rückstände des Elements auf dem Substrat bleiben. Bevorzugt ist das Substrat ausgewählt aus der Gruppe bestehen aus einem Polymer, einem Glas, einer Keramik, einem Papier, einem beschichteten Papier, einem Holz, einem Metall oder einer Kombination hiervon.

Das Aushärtung der Polyurethan-Prepolymer-Medium-Mischung, insbesondere der Polyurethan-Prepolymer-Wasser-Mischung, aus Schritt II) in Schritt III) unter Erhalt des visko-elastischen Elements kann bei jeder Bedingung erfolgen, die der Fachmann hierfür auswählen würde. Bevorzugt findet das Aushärten bei einer Temperatur in einem Bereich von 20 bis 100 °C, oder bevorzugt in einem Bereich von 30 bis 80 °C, oder bevorzugt in einem Bereich von 40 bis 70 °C statt. Bevorzugt findet das Aushärten über einen Zeitraum in einem Bereich von 1 bis 180 Minuten, oder bevorzugt in einem Bereich von 2 bis 100 Minuten, oder bevorzugt in einem Bereich von 5 bis 60 Minuten, oder bevorzugt in einem Bereich von 7 bis 30 Minuten statt. Bevorzugt findet das Aushärten bei einer Temperatur in einem Bereich von 40 bis 80 °C für einen Zeitraum von 2 bis 30 Minuten statt. Die Temperatur wird bevorzugt mittels eines Heißluftofen, eines Föns, eines geheizten Substrats, eines Strahler, beispielsweise eines IR-Strahlers oder einer Kombination hieraus statt.

Besonders bevorzugt handelt es sich bei dem Medium um Wasser. Wasser bildet im Allgemeinen, neben wahlweise vorhandenen anderen flüssigen Medien, wie beispielsweise Lösungsmitteln, den Hauptbestandteil (≥ 50 Gew.-%) des Mediums, bezogen auf die Gesamtmenge der Polyurethan-Prepolymer-Medium-Mischung, gegebenenfalls auch das alleinige flüssig enthaltene Medium.

Ein weiterer Gegenstand der Erfindung betrifft einen Gehörschutzstöpsel zum Einführen in ein Ohr oder einen Gehörgang, beinhaltend ein erfindungsgemäßes visko-elastisches Element oder ein visko-elastischen Element, das gemäß dem erfindungsgemäßen Verfahren hergestellt wurde.

Ein weiterer Gegenstand der Erfindung betrifft die Verwendung des zuvor beschriebenen erfindungsgemäßen visko-elastischen Elements sowie des gemäß dem erfindungsgemäßen Verfahren hergestellten visko-elastischen Elements zur Geräuschminimierung in einem Innenraum. Als Innenraum kann dabei jedes von mindestens einer Wand umgebene Volumen verstanden werden. Geeignet als Innenraum ist jedes Volumen, das der Fachmann hierfür auswählen würde. Beispiele für einen Innenraum ist mindestens ein Teil eines Autoinnenraums, mindestens ein Teil eines Flugzeuginnenraums, ein Gehörgang bzw. Ohrinnenraum, ein Bett bzw. Bettkasten, ein Stuhl bzw. Stuhlgestell, ein Kissen bzw. Kissenbezug oder eine Kombination aus mindestens zwei hiervon.

Bevorzugt ist die Verwendung des zuvor beschriebenen erfindungsgemäßen visko-elastischen Elements sowie des gemäß dem erfindungsgemäßen Verfahren hergestellten visko-elastischen Elements zur Minderung von Druckstellen an Körperteilen. Beispiele hierfür sind visko-elastische Elemente in Form von Matratzen, Kissen, Stuhlpolster, Sesselpolster, Autositze, Flugzeugsitze oder Kombinationen von mindestens zwei hiervon, die bevorzugt dazu verwendet werden können mindestens ein Körperteil über einen Zeitraum von mehreren Stunden, oder bevorzugt von mehreren Tagen, oder bevorzugt von mehreren Wochen zu lagern ohne dass die gelagerten Körperteile Druckstellen entwickeln.

Ein weiterer Gegenstand der Erfindung betrifft ein Kit zum Herstellen eines visko-elastischen Elements, bestehend aus
- (V1): Einem isocyanatfunktionellen Polyurethan-Prepolymer;
- (V2): Einer Polyurethanharnstoff-Dispersion.

Sämtliche Ausführungen, die zuvor für die Komponenten (V1) und (V2) im Zusammenhang mit dem erfindungsgemäßen visko-elastischen Element gemacht wurden, sind ebenfalls für die Komponenten (V1) und (V2) des Kits gültig.

In einer bevorzugten Ausführungsform des Kits liegt die Komponente (V2) als wässrige Dispersion vor. Was unter einer wässrigen Dispersion zu verstehen ist, ist bereits zu dem erfindungsgemäßen visko-elastischen Element beschrieben und für das Kit ebenfalls gültig.

In einer bevorzugten Ausführungsform des Kits ist Komponente (V1) erhältlich durch Reaktion von mindestens den Komponenten
- V1A): aliphatischen Diisocyanaten mit
- V1B): di- bis hexafunktionellen Polyalkylenoxiden mit einem Ethylenoxidanteil von 50 bis 100 mol%, bezogen auf die Gesamtmenge der enthaltenen Oxyalkylengruppen.

In einer bevorzugten Ausführungsform des Kit ist der Polyurethanharnstoff der Polyurethanharnstoff-Dispersion V2) erhältlich durch Umsetzung von wenigstens
- V2A): einer aliphatischen Polyisocyanat-Komponente mit einer mittleren Isocyanatfunktionalität von ≥ 1,8 und ≤ 2,6,
- V2B): einer polymeren Polyether-Polyol-Komponente,
- V2C): einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
- V2D): gegebenenfalls weiteren hydrophilierenden Komponenten, die unterschiedlich sind von C2) oder
- V2E): gegebenenfalls hydroxyfunktionellen Verbindungen mit einem Molekulargewicht von 62 bis 399 g/mol oder
- V2F): gegebenenfalls mindestens einem weiteren polymeren Polyol, welches unterschiedlich ist von V2B) oder
- V2G): einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und gegebenenfalls einer aliphatischen Polyisocyanat-Komponente mit einer mittleren Isocyanatfunktionalität von ≥ 2,6 und ≤ 4.

### Methoden:

Sofern nicht abweichend gekennzeichnet, beziehen sich alle Prozentangaben auf das Gesamtgewicht der Zusammensetzungen.

Sofern nicht abweichend vermerkt, beziehen sich alle analytischen Messungen auf Messungen bei Temperaturen von 23 °C (auch als Raumtemperatur RT bezeichnet) und Normaldruck.

### Dickenmessung:

Die Schichtdickenbestimmung wurde mit einem Drucklufttaster und zur Ausgabe der Schichtdicke angeschlossenem Display der Firma Heidehain (MT25P) ermittelt.

### Dichtemessung:

Zur Dichtebestimmung wurde ein Probenstück mit Hilfe eines Stanzeisens in der Dimension 5 × 5 cm² (mit abgerundeten Ecken und Kurvenradius von 3 mm) ausgestanzt. Die Höhe wurde aus dem Mittelwert einer 5fach-Bestimmung mittels oben beschriebener Methode ermittelt. Zur anschließenden Berechnung der Dichte wurde die Masse des Probenstückes an einer Mettler Toledo XS603S Waage bestimmt.

### Retention:

Die maximale Absorption des Schaumes wurde gemäß DIN EN 13726-1:2002-06 an einem Schaumstück der Größe 5 × 5 cm bestimmt. Das Schaumstück der Größe 5 × 5 cm wird nach vollständiger Absorption der Prüflösung A, (hergestellt nach DIN EN 13726-1:2002-06) mit einer Pinzette angehoben und für 30 Sekunden abtropfen gelassen und gewogen.

Das Schaumstück wird auf ein Metallpodest gelegt und mit einem Gewicht von 6 kg für 20 Sekunden gleichmäßig beschwert. Das Gewicht wird entfernt und nach Entfernen des Gewichtes wird das Schaumstück erneut gewogen. Die verbliebene Feuchtigkeitsmenge wird im Vergleich prozentual zur maximalen Absorption bestimmt.

### Bestimmung der Rückstellzeit:

Ein zylindrisches Schaumstück mit den Maßen 6 cm × 7 cm (Durchmesser × Höhe) wurde mit einem Gewicht von 2 kg (entspricht einem Druck von 7 kPa) beschwert. Nach der Kompression bis ungefähr 50% und anschließender Entfernung des Gewichts wurde die Zeit ermittelt bis der Schaumzylinder mindestens 90% seiner Ursprungshöhe wieder erreicht hat.

### Herstellung eines erfindungsgemäßen visko-elastischen Schaums :

### Beispiel 1 (B1): Klebstofffreier Wundkontaktschaum

125 g Baymedix^{®} FP505 (V1), erhältlich bei Covestro Deutschland AG, wurden mit 75 g einer wässrigen Lösung aus 0,44 Gew.-% Natriumhydrogencarbonat (erhältlich bei Fluka, Deutschland), 1,6 Gew.-% Pluronic^{®} PE6800 (erhältlich bei BASF SE, Deutschland), 0,13 Gew.-% Zitronensäure-Monohydrat (erhältlich bei ACROS Organics) und 66,6 Gew.% Baymedix^{®} AD111 (V2) (erhältlich bei Covstro) unter starkem Rühren für 7s mit Hilfe eines Rührwerks mit Ankerrührblatt (Labordissolver 5 green 037 der Firma Pendraulik GmbH) bei 930 U/min. vermischt und danach auf ein Trennpapier des Typs Y 05200 der Felix Schöller Group, Osnabrück mittels eines Rakels von 1,5 mm Spalt in x-y Richtung aufgebracht. Danach wurde die Oberfläche der Reaktionsmischung, die sich in x-y Richtung erstreckt, mit einer genadelten Variante (umfassend Löcher mit einem Durchmesser von 0,1 mm² und einer Locherdichte von 10 pro cm²) desselben Papiers abgedeckt.

Folgende Messergebnisse wurden ermittelt:

| **Messmethode:** | **Einheit** | **Ergebnis:** |
|---|---|---|
| Dichte | g/l | 277 |
| Absorption | % | 1080 |
| Retention | % | 55 |
| Bruchspannung | MPa | 0,19 |
| Bruchdehnung | % | 255 |
| E-Modul | MPa | 0,094 |
| Rückstellzeit | s | 13 |
| Verlustfaktor (tan δ) bei 1Hz | - | 0,286 |
| Dämpfung (aus Stauchhärte) | - | 0,52 |

### Beispiel 2 (B2) Klebstofffreier Wundkontaktschaum

125 g Baymedix^{®} FP505 (V1), erhältlich bei Covestro Deutschland AG, wurden mit 50 g einer wässrigen Lösung aus 0,66 Gew.-% Natriumhydrogencarbonat (erhältlich bei Fluka, Deutschland), 2,4 Gew.-% Pluronic^{®} PE6800 (erhältlich bei BASF SE, Deutschland), 0,2 Gew.-% Zitronensäure-Monohydrat (erhältlich bei ACROS Organics) und 50,0 Gew.-% Baymedix^{®} AD111 (V2) (erhältlich bei Covstro) unter starkem Rühren für 7s mit Hilfe eines Rührwerks mit Ankerrührblatt (Labordissolver 5 green 037 der Firma Pendraulik GmbH) bei 930 U/min. vermischt und danach auf ein Trennpapier des Typs Y 05200 der Felix Schöller Group, Osnabrück mittels eines Rakels von 1,5 mm Spalt in x-y Richtung aufgebracht. Danach wurde die Oberfläche der Reaktionsmischung, die sich in x-y Richtung erstreckt, mit einer genadelten Variante (umfassend Löcher mit einem Durchmesser von 0,1 mm² und einer Lochdichte von 10 pro cm²) des gleichen Papiers abgedeckt.

Folgende Messergebnisse wurden ermittelt:

| **Messmethode:** | **Einheit** | **Ergebnis:** |
|---|---|---|
| Dichte | g/l | 210 |
| Absorption | % | 1409 |
| Retention | % | 46 |
| Bruchspannung | MPa | 220 |
| Bruchdehnung | % | 0,14 |
| E-Modul | MPa | 0,096 |
| Rückstellzeit | s | 4 |
| Verlustfaktor (tan δ) bei 1Hz | - | 0,187 |
| Dämpfung (aus Stauchhärte) | - | 0,47 |

### Vergleichsbeispiel (VB1) (nicht-erfindungsgemäß):

125 g Baymedix^{®} FP505 (V1), erhältlich bei Covestro Deutschland AG, wurden mit 25 g einer wässrigen Lösung aus 1,32 Gew.-% Natriumhydrogencarbonat (erhältlich bei Fluka, Deutschland), 4,8 Gew.-% Pluronic^{®} PE6800 (erhältlich bei BASF SE, Deutschland), 0,4 Gew.-% Zitronensäure-Monohydrat (erhältlich bei ACROS Organics, Belgien) unter starkem Rühren für 7s mit Hilfe eines Rührwerks mit Ankerrührblatt (Labordissolver 5 green 037 der Firma Pendraulik GmbH) bei 930 U/min. vermischt und danach auf ein Trennpapier des Typs Y 05200 der Felix Schöller Group, Osnabrück mittels eines Rakels von 1,5 mm Spalt in x-y Richtung aufgebracht. Danach wurde die Oberfläche der Reaktionsmischung, die sich in x-y Richtung erstreckt, mit einer genadelten Variante (umfassend Löcher mit einem Durchmesser von 0,1 mm² und einer Lochdichte von 10 pro cm²) des gleichen Papiers abgedeckt.

| **Messmethode:** | **Einheit** | **Ergebnis:** |
|---|---|---|
| Dichte | g/l | 133 |
| Absorption | % | 1874 |
| Retention | % | 71 |
| Bruchspannung | MPa | 0,09 |
| Bruchdehnung | % | 129 |
| E-Modul | MPa | 0,078 |
| Rückstellzeit | s | 1 |
| Verlustfaktor (tan δ) bei 1Hz | - | 0,137 |
| Dämpfung (aus Stauchhärte) | - | 0,35 |

### Vergleichsbeispiel (VB2) (nicht-erfindungsgemäß):

125 g Prepolymergemischs aus Patent BMS111012 (Beispiel 1) wurden mit 22,5 g einer wässrigen Lösung aus 2,6 Gew.-% Natriumoleat (erhältlich bei abcr Gmbh, Deutschland) und 55,6 Gew.-% Baymedix^{®} FD10, erhältlich bei Covestro Deutschland AG, 3 unter heftigem Rühren für 7s mit Hilfe eines Rührwerks mit Ankerrührblatt (Labordissolver 5 green 037 der Firma Pendraulik GmbH) bei 930 U/min. vermischt und danach auf ein Trennpapier des Typs Y 05200 der Felix Schöller Group, Osnabrück mittels eines Rakels von 1,5 mm Spalt in x-y Richtung aufgebracht. Danach wurde die Oberfläche der Reaktionsmischung, die sich in x-y Richtung erstreckt, mit einer genadelten Variante, wie in (B1) beschrieben, des gleichen Papiers abgedeckt.

| **Messmethode:** | **Einheit** | **Ergebnis:** |
|---|---|---|
| Dichte | g/l | Nicht bestimmt (n.b.) |
| Absorption | % | n.b. |
| Retention | % | n.b. |
| Bruchspannung | MPa | n.b. |
| Bruchdehnung | % | n.b. |
| E-Modul | MPa | n.b. |
| Rückstellzeit | s | 1 |
| Verlustfaktor (tan δ) bei 1Hz | - | n.b. |
| Dämpfung (aus Stauchhärte) | - | 0,26 |

### Vergleichsbeispiel (VB3) (nicht-erfindungsgemäß):

125 g Prepolymergemischs aus Patent BMS111012 (Beispiel 2) wurden mit 55,8 g einer wässrigen Lösung aus 1,1 Gew.-% Natriumoleat (erhältlich bei abcr Gmbh, Deutschland) und 78,5 Gew.-% Baymedix^{®} FD103, erhältlich bei Covestro Deutschland AG, unter heftigem Rühren für 7s mit Hilfe eines Rührwerks mit Ankerrührblatt (Labordissolver 5 green 037 der Firma Pendraulik GmbH) bei 930 U/min. vermischt und danach auf ein Trennpapier des Typs Y 05200 der Felix Schöller Group, Osnabrück mittels eines Rakels von 1,5 mm Spalt in x-y Richtung aufgebracht. Danach wurde die Oberfläche der Reaktionsmischung, die sich in x-y Richtung erstreckt, mit einer genadelten Variante, wie in (B1) beschrieben, des gleichen Papiers abgedeckt.

| **Messmethode:** | **Einheit** | **Ergebnis:** |
|---|---|---|
| Dichte | g/l | n.b. |
| Absorption | % | n.b. |
| Retention | % | n.b. |
| Bruchspannung | MPa | n.b. |
| Bruchdehnung | % | n.b. |
| E-Modul | MPa | n.b. |
| Rückstellzeit | s | 1 |
| Verlustfaktor (tan δ) bei 1Hz | - | n.b. |
| Dämpfung (aus Stauchhärte) | - | 0,26 |

## Patentansprüche

1. Ein visko-elastisches Element beinhaltend einen Polyurethan-Schaum, erhältlich durch Reaktion von mindestens den Komponenten
(V1) einem isocyanatfunktionellen Prepolymer, erhältlich durch Umsetzung von mindestens den Komponenten
V1A) aliphatischen Diisocyanaten mit
V1B) di- bis hexafunktionellen Polyalkylenoxiden mit einem Ethylenoxidanteil von 50 bis 100 mol%, bezogen auf die Gesamtmenge der enthaltenen Oxyalkylengruppen, in Anwesenheit von
(V2) einer Polyurethanharnstoff-Dispersion, wobei der Polyurethanharnstoff erhältlich ist durch Umsetzung von wenigstens
V2A) einer aliphatischen Polyisocyanat-Komponente mit einer mittleren Isocyanatfunktionalität von ≥ 1,8 und ≤ 2,6;
V2B) einer polymeren Polyether-Polyol-Komponente;
V2C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist;
V2D) gegebenenfalls weiteren hydrophilierenden Komponenten, die unterschiedlich sind von C2),
V2E) gegebenenfalls hydroxyfunktionellen Verbindungen mit einem Molekulargewicht von 62 bis 399 g/mol,
V2F) gegebenenfalls mindestens einem weiteren polymeren Polyol, welches unterschiedlich ist von V2B),
V2G) einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
V2H) gegebenenfalls einer aliphatischen Polyisocyanat-Komponente mit einer mittleren Isocyanatfunktionalität von ≥ 2,6 und ≤ 4,
wobei die Reaktion des Prepolymers (V1) in Anwesenheit des Polyurethanharnstoff (V2) mit einem isocyanatreaktive-Gruppen enthaltenden Medium stattfindet.

2. Das visko-elastische Element gemäß Anspruch 1, wobei die Komponenten (V1) mit (V2) bei der Reaktion der Komponenten (V1) und (V2) in einem Gewichtsverhältnis von 5:1 bis 1:1 liegt, bezogen auf das Gesamtgewicht der beiden Komponenten (V1) und (V2).

3. Das visko-elastische Element gemäß einem der Ansprüche 1 oder 2, wobei das visko-elastische Element mindestens eine der folgenden Eigenschaften aufweist:
i. eine Dämpfung von 0,40 bis 0,90 aufweist, gemessen nach DIN EN ISO 3386-1:2015-10;
ii. eine Bruchspannung in einem Bereich von 0,12 MPa bis 0,40 MPa gemessen nach DIN EN ISO 527-2;
iii. eine Bruchdehnung in einem Bereich von 150 % bis 300% gemessen nach DIN EN ISO 527-2;
iv. eine Absorption von Wasser von ≤ 1800 % (g/g), oder bevorzugt von ≤ 1700 % (g/g), oder bevorzugt von ≤ 1500 % (g/g), bezogen auf Gewicht des trockenen Schaums gemessen nach DIN EN 13726-1;
v. eine Retention von Wasser von ≤ 80 %, oder bevorzugt von ≤ 70 %, oder bevorzugt von ≤ 60 %, bezogen auf die maximale Absorption;
vi. eine Rückstellzeit nach Kompression des Ursprungvolumens um 50 % (bei einem Druck von 7 kPa) auf mindestens 90 % des Ursprungsvolumens bei Normaldruck und RT in einem Bereich von 2 bis 50 Sekunden;
vii. ein Volumen in einem Bereich von 0,1 cm³ bis 1 m³;
viii. eine Dichte in einem Bereich 150 bis 400 g/l;
ix. einen Verlustfaktor ≥ 0,140, bevorzugt einen Verlustfaktor in einem Bereich von 0,140 bis 0,400 aufweist, gemessen nach DIN EN ISO 6721-1
x. einen E-Modul nach DIN EN ISO 527-2 in einem Bereich von 0,08 MPa bis 0,25 MPa, oder bevorzugt von 0,08 MPa bis 0,2 MPa, oder bevorzugt von 0,08 MPa bis 0,15 MPa.

4. Das visko-elastische Element gemäß einem der vorhergehenden Ansprüche, **dadurch gekennzeichnet, dass** die Komponenten V2B) und V2F) zusammen ≤ 30 Gew.-% an Komponente V2F), bezogen auf die Gesamtmasse der Komponenten V2B) und V2F) enthalten.

5. Das visko-elastische Element gemäß einem der vorhergehenden Ansprüche, wobei die Komponente V2A) Isophorondiisocyanat und/oder Hexamethylendiisocyanat ist.

6. Das visko-elastische Element gemäß einem der vorhergehenden Ansprüche, **dadurch gekennzeichnet, dass** die Komponente V2B) Poly(tetramethylenglykol)polyetherpolyole enthält oder daraus besteht.

7. Das visko-elastische Element gemäß einem der vorhergehenden Ansprüche, **dadurch gekennzeichnet, dass** die Komponente (V1) ein isocyanatfunktionelles Prepolymer mit einem Gewichtsanteil an niedermolekularen, aliphatischen Diisocyanaten mit einer Molmasse von ≥ 140 bis ≤ 278 g/mol von ≤ 1.0 Gew.-%, bezogen auf das Prepolymer ist, erhältlich durch Umsetzung von
V1A) niedermolekularen, aliphatischen Diisocyanaten einer Molmasse von ≥ 140 bis ≤ 278 g/mol, bevorzugt ≥ 168 bis ≤ 258 g/mol mit
V1B) di- bis hexafunktionellen Polyalkylenoxiden einer OH-Zahl von ≥ 22,5 bis ≤ 112 mg KOH/g und einem Ethylenoxidanteil von ≥ 50 bis ≤ 100 mol%, bezogen auf die Gesamtmenge der enthaltenen Oxyalkylengruppen;
V1C) gegebenenfalls heterocyclische, 4-Ring oder 6-Ring-Oligomere von niedermolekularen, aliphatischen Diisocyanaten mit einer Molmasse von ≥ 140 bis ≤ 278 g/mol, bevorzugt ≥ 168 bis ≤ 258 g/mol;
V1D) gegebenenfalls Katalysatoren;
V1E) gegebenenfalls Alkalimetallsalze schwacher anorganischer Säuren;
V1F) gegebenenfalls Tenside;
V1G) gegebenenfalls ein- oder mehrwertige Alkohole;
V1H) die folgenden Komponenten:
V1H1) ein oder mehrere niedermolekulare, aliphatische Diisocyanate einer Molmasse von ≥ 140 bis ≤ 278 g/mol, bevorzugt ≥ 168 bis ≤ 258 g/mol und/oder daraus herstellbare Polyisocyanate mit einer Isocyanatfunktionalität von ≥ 2 bis ≤ 6;
und/oder
V1H2) ein oder mehrere monofunktionelle Polyalkylenoxide mit einer OH-Zahl von ≥ 10 bis ≤ 250 und einem Ethylenoxidanteil von ≥ 50 bis ≤ 100 mol%, bezogen auf die Gesamtmenge der enthaltenen Oxyalkylengruppen;
und/oder
V1H3) eine hydrophile Isocyanatkomponente, welche durch die Umsetzung von unter V1H1) genannten Komponenten mit unter V1H2) genannten Komponenten erhältlich ist.

8. Ein Verfahren zur Herstellung eines visko-elastischen Elements umfassend die Schritte:
I) Mischen eines isocyanatfunktionellen Polyurethan-Prepolymers (V1) mit mindestens einer Polyurethanharnstoff-Dispersion (V2) in Anwesenheit eines isocyanatreaktive-Gruppen enthaltenden Mediums unter Erhalt einer Polyurethan-Prepolymer-Medium-Mischung, wobei das Medium bevorzugt Wasser ist,
II) Aufbringen der Polyurethan-Prepolymer-Medium-Mischung aus Schritt I) auf ein Substrat,
III) Aushärtung der Polyurethan-Prepolymer-Medium-Mischung, insbesondere einer Polyurethan-Prepolymer-Wasser-Mischung, aus Schritt II) unter Erhalt des visko-elastischen Elements,
IV) ggf. Entfernen des visko-elastischen Elements von dem Substrat.

9. Das Verfahren gemäß Anspruch 8, wobei das Mischen in Schritt I) innerhalb von 3 bis 90 Sekunden erfolgt, bevor die Mischung in Schritt II) auf das Substrat aufgebracht wird.

10. Ein Gehörschutzstöpsel zum Einführen in ein Ohr, beinhaltend ein visko-elastisches Element gemäß einem der Ansprüche 1 bis 7 oder hergestellt nach einem der Ansprüche 8 bis 9.

11. Verwendung eines visko-elastischen Elements gemäß einem der Ansprüche 1 bis 7, hergestellt nach einem Verfahren gemäß einem der Ansprüche 8 bis 9 zur Geräuschminimierung in einem Innenraum.

12. Ein Kit zum Herstellen eines visko-elastischen Elements, bestehend aus
(V1) Einem isocyanatfunktionellen Polyurethan-Prepolymer;
(V2) Einer Polyurethanharnstoff-Dispersion.

13. Das Kit gemäß Anspruch 12, wobei die Komponente (V2) als wässrige Dispersion vorliegt.

14. Das Kit gemäß Anspruch 12 oder 13, wobei Komponente (V1) erhältlich ist durch Reaktion von mindestens den Komponenten
V1A) aliphatischen Diisocyanaten mit
V1B) di- bis hexafunktionellen Polyalkylenoxiden mit einem Ethylenoxidanteil von 50 bis 100 mol%, bezogen auf die Gesamtmenge der enthaltenen Oxyalkylengruppen.

15. Der Kit nach einem der Ansprüche 12 bis 14, wobei der Polyurethanharnstoff der Polyurethanharnstoff-Dispersion V2) erhältlich ist durch Umsetzung von wenigstens
V2A) einer aliphatischen Polyisocyanat-Komponente mit einer mittleren Isocyanatfunktionalität von ≥ 1,8 und ≤ 2,6,
V2B) einer polymeren Polyether-Polyol-Komponente,
V2C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
V2D) gegebenenfalls weiteren hydrophilierenden Komponenten, die unterschiedlich sind von C2),
V2E) gegebenenfalls hydroxyfunktionellen Verbindungen mit einem Molekulargewicht von 62 bis 399 g/mol,
V2F) gegebenenfalls mindestens einem weiteren polymeren Polyol, welches unterschiedlich ist von V2B),
V2G) einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und gegebenenfalls einer aliphatischen Polyisocyanat-Komponente mit einer mittleren Isocyanatfunktionalität von ≥ 2,6 und ≤ 4.

## Claims

1. Viscoelastic element including a polyurethane foam, obtainable by reaction of at least the following components:
(V1) an isocyanate-functional prepolymer obtainable by reaction of at least the following components:
V1A) aliphatic diisocyanates with
V1B) di- to hexafunctional polyalkylene oxides having an ethylene oxide content of 50 to 100 mol%, based on the total amount of oxyalkylene groups present,
in the presence of
(V2) a polyurethaneurea dispersion, where the polyurethaneurea is obtainable by reacting at least V2A) an aliphatic polyisocyanate component having an average isocyanate functionality of ≥ 1.8 and ≤ 2.6;
V2B) a polymeric polyether polyol component;
V2C) an amino-functional chain extender component having at least 2 isocyanate-reactive amino groups, containing at least one amino-functional compound C1) that does not have any ionic or ionogenic groups and/or an amino-functional compound C2) that has ionic or ionogenic groups;
V2D) optionally further hydrophilizing components different than C2),
V2E) optionally hydroxy-functional compounds having a molecular weight of 62 to 399 g/mol,
V2F) optionally at least one further polymeric polyol which is different than V2B),
V2G) a compound having exactly one isocyanate-reactive group or a compound having more than one isocyanate-reactive group, where only one of the isocyanate-reactive groups reacts with the isocyanate groups present in the reaction mixture under the reaction conditions chosen, and
V2H) optionally an aliphatic polyisocyanate component having an average isocyanate functionality of ≥ 2.6 and ≤ 4,
wherein the reaction of the prepolymer (V1) in the presence of the polyurethaneurea (V2) takes place with a medium containing isocyanate-reactive groups.

2. Viscoelastic element according to Claim 1, wherein components (V1) with (V2) are in a weight ratio of 5:1 to 1:1 in the reaction of components (V1) and (V2), based on the total weight of the two components (V1) and (V2).

3. Viscoelastic element according to either of Claims 1 and 2, wherein the viscoelastic element has at least one of the following properties:
i. damping of 0.40 to 0.90, measured to DIN EN ISO 3386-1:2015-10;
ii. breaking stress within a range from 0.12 MPa to 0.40 MPa, measured to DIN EN ISO 527-2;
iii. elongation at break within a range from 150% to 300%, measured to DIN EN ISO 527-2;
iv. water absorption of ≤ 1800% (g/g), or preferably of ≤ 1700% (g/g), or preferably of ≤ 1500% (g/g), based on weight of the dry foam, measured to DIN EN 13726-1;
v. water retention of ≤ 80%, or preferably of ≤ 70%, or preferably of ≤ 60%, based on the maximum absorption;
vi. recovery time after compression of the original volume by 50% (at a pressure of 7 kPa) to at least 90% of the original volume at standard pressure and RT within a range from 2 to 50 seconds;
vii. volume within a range from 0.1 cm³ to 1 m³;
viii. density within a range of 150 to 400 g/l;
ix. loss factor ≥ 0.140, preferably a loss factor within a range from 0.140 to 0.400, measured to DIN EN ISO 6721-1
x. modulus of elasticity to DIN EN ISO 527-2 within a range from 0.08 MPa to 0.25 MPa, or preferably from 0.08 MPa to 0.2 MPa, or preferably from 0.08 MPa to 0.15 MPa.

4. Viscoelastic element according to any of the preceding claims, **characterized in that** components V2B) and V2F) together contain ≤ 30% by weight of component V2F), based on the total mass of components V2B) and V2F).

5. Viscoelastic element according to any of the preceding claims, wherein component V2A) is isophorone diisocyanate and/or hexamethylene diisocyanate.

6. Viscoelastic element according to any of the preceding claims, **characterized in that** component V2B) comprises or consists of poly(tetramethylene glycol) polyether polyols.

7. Viscoelastic element according to any of the preceding claims, **characterized in that** component (V1) is an isocyanate-functional prepolymer having a proportion by weight of low molecular weight, aliphatic diisocyanates having a molar mass of ≥ 140 to ≤ 278 g/mol of ≤ 1.0% by weight, based on the prepolymer, obtainable by reaction of
V1A) low molecular weight, aliphatic diisocyanates of molar mass from ≥ 140 to ≤ 278 g/mol, preferably ≥ 168 to ≤ 258 g/mol, with
V1B) di- to hexafunctional polyalkylene oxides of OH number from ≥ 22.5 to ≤ 112 mg KOH/g and of ethylene oxide content from ≥ 50 to ≤ 100 mol%, based on the total amount of oxyalkylene groups present;
V1C) optionally heterocyclic 4-membered or 6-membered ring oligomers of low molecular weight, aliphatic diisocyanates having a molar mass of ≥ 140 to ≤ 278 g/mol, preferably ≥ 168 to ≤ 258 g/mol;
V1D) optionally catalysts;
V1E) optionally alkali metal salts of weak inorganic acids;
V1F) optionally surfactants;
V1G) optionally mono- or polyhydric alcohols;
V1H) the following components:
V1H1) one or more low molecular weight, aliphatic diisocyanates having a molar mass of ≥ 140 to ≤ 278 g/mol, preferably ≥ 168 to ≤ 258 g/mol and/or polyisocyanates preparable therefrom that have an isocyanate functionality of ≥ 2 to ≤ 6;
and/or
V1H2) one or more monofunctional polyalkylene oxides having an OH number of ≥ 10 to ≤ 250 and an ethylene oxide content of ≥ 50 to ≤ 100 mol%, based on the total amount of the oxyalkylene groups present;
and/or
V1H3) a hydrophilic isocyanate component obtainable by the reaction of components mentioned under V1H1) with components mentioned under V1H2).

8. Process for producing a viscoelastic element, comprising the steps of:
I) mixing an isocyanate-functional polyurethane prepolymer (V1) with at least one polyurethaneurea dispersion (V2) in the presence of a medium containing isocyanate-reactive groups to obtain a polyurethane prepolymer/medium mixture, the medium preferably being water,
II) applying the polyurethane prepolymer/medium mixture from step I) to a substrate,
III) curing the polyurethane prepolymer/medium mixture, especially a polyurethane prepolymer/water mixture, from step II) to obtain the viscoelastic element,
IV) optionally removing the viscoelastic element from the substrate.

9. Process according to Claim 8, wherein the mixing in step I) is effected within 3 to 90 seconds before the mixture is applied to the substrate in step II).

10. Earplug for insertion into an ear, comprising a viscoelastic element according to any of Claims 1 to 7 or produced according to any of Claims 8 to 9.

11. Use of a viscoelastic element according to any of Claims 1 to 7, produced by a process according to either of Claims 8 and 9, for minimization of noise in an interior.

12. Kit for producing a viscoelastic element, consisting of
(V1) an isocyanate-functional polyurethane prepolymer;
(V2) a polyurethaneurea dispersion.

13. Kit according to Claim 12, wherein component (V2) is in the form of an aqueous dispersion.

14. Kit according to Claim 12 or 13, wherein component (V1) is obtainable by reaction of at least the following components:
V1A) aliphatic diisocyanates with
V1B) di- to hexafunctional polyalkylene oxides having an ethylene oxide content of 50 to 100 mol%, based on the total amount of the oxyalkylene groups present.

15. Kit according to any of Claims 12 to 14, wherein the polyurethaneurea of the polyurethaneurea dispersion V2) is obtainable by reacting at least
V2A) an aliphatic polyisocyanate component having an average isocyanate functionality of ≥ 1.8 and ≤ 2.6, V2B) a polymeric polyether polyol component,
V2C) an amino-functional chain extender component having at least 2 isocyanate-reactive amino groups, containing at least one amino-functional compound C1) that does not have any ionic or ionogenic groups and/or an amino-functional compound C2) that has ionic or ionogenic groups,
V2D) optionally further hydrophilizing components different than C2),
V2E) optionally hydroxy-functional compounds having a molecular weight of 62 to 399 g/mol,
V2F) optionally at least one further polymeric polyol different than V2B),
V2G) a compound having exactly one isocyanate-reactive group or a compound having more than one isocyanate-reactive group, where only one of the isocyanate-reactive groups reacts with the isocyanate groups present in the reaction mixture under the reaction conditions chosen, and optionally an aliphatic polyisocyanate component having an average isocyanate functionality of ≥ 2.6 and ≤ 4.

## Revendications

1. Élément viscoélastique comportant une mousse de polyuréthane pouvant être obtenue par réaction d'au moins les composants
(V1) un prépolymère à fonction isocyanate, pouvant être obtenu par mise en réaction d'au moins les composants
V1A) diisocyanates aliphatiques avec
V1B) des polyoxyalkylènes di- à hexafonctionnels ayant une proportion d'oxyde d'éthylène de 50 à 100 % en moles, par rapport à la quantité totale des groupes oxyalkylène contenus,
en présence de
(V2) une dispersion de polyuréthane-urée, la polyuréthane-urée pouvant être obtenue par mise en réaction d'au moins
V2A) un composant polyisocyanate aliphatique ayant une fonctionnalité isocyanate moyenne de ≥ 1,8 et ≤ 2,6 ;
V2B) un composant polymère polyéther-polyol ;
V2C) un composant extendeur de chaîne à fonction amino, comportant au moins 2 groupes amino réactifs avec des isocyanates, contenant au moins un composé C1) à fonction amino, qui ne comporte pas de groupes ioniques ou ionogènes et/ou un composé C2) à fonction amino, qui comporte des groupes ioniques ou ionogènes ;
V2D) éventuellement des composants hydrophilisants supplémentaires qui sont différents de C2),
V2E) éventuellement des composés à fonction hydroxy ayant une masse moléculaire de 62 à 399 g/moles,
V2F) éventuellement au moins un polyol polymère supplémentaire qui est différent de V2B),
V2G) un composé qui comporte exactement un groupe réactif avec un isocyanate, ou un composé qui comporte plus d'un groupe réactif avec un isocyanate, un seul des groupes réactifs avec des isocyanates réagissant, dans les conditions réactionnelles choisies, avec les groupes isocyanate présents dans le mélange réactionnel et
V2H) éventuellement un composant polyisocyanate aliphatique ayant une fonctionnalité isocyanate moyenne de ≥ 2,6 et ≤ 4,
dans lequel la réaction du prépolymère (V1) a lieu en présence de la polyuréthane-urée (V2) avec un milieu contenant des groupes réactifs avec des isocyanates.

2. Élément viscoélastique selon la revendication 1, dans lequel, dans la réaction des composants (V1) et (V2) le composant (V1) se trouve avec le composant (V2) en un rapport pondéral de 5:1 à 1:1, par rapport au poids total des deux composants (V1) et (V2).

3. Élément viscoélastique selon l'une quelconque des revendications 1 et 2, dans lequel l'élément viscoélastique présente au moins une des propriétés suivantes :
i. un amortissement de 0,40 à 0,90, mesuré selon DIN EN ISO 3386-1:2015-10 ;
ii. une contrainte de rupture dans une plage de 0,12 MPa à 0,40 MPa, mesurée selon DIN EN ISO 527-2 ;
iii. un allongement à la rupture dans une plage de 150 % à 300 %, mesuré selon DIN EN ISO 527-2 ;
iv. une absorption d'eau de ≤ 1 800 % (g/g), ou de préférence de ≤ 1 700 % (g/g), ou de préférence de ≤ 1 500 % (g/g) par rapport au poids de la mousse sèche, mesurée selon DIN EN 13726-1 ;
v. une rétention d'eau de ≤ 80 %, ou de préférence de ≤ 70 %, ou de préférence de ≤ 60 %, par rapport à l'absorption maximale ;
vi. un temps de rétablissement après compression du volume initial de 50 % (sous une pression de 7 kPa) à au moins 90 % du volume initial sous la pression normale et à la TA, dans un intervalle de 2 à 50 secondes ;
vii. un volume dans une plage de 0,1 cm³ à 1 m³ ;
viii. une densité dans une plage de 150 à 400 g/l ;
ix. un facteur de perte ≥ 0,140, de préférence un facteur de perte dans une plage de 0,140 à 0,400, mesuré selon DIN EN ISO 6721-1 ;
x. un module d'élasticité selon DIN EN ISO 527-2 dans une plage de 0,08 MPa à 0,25 MPa, ou de préférence de 0,08 MPa à 0,2 MPa, ou de préférence de 0,08 MPa à 0,15 MPa.

4. Élément viscoélastique selon l'une quelconque des revendications précédentes, **caractérisé en ce que** les composants V2B) et V2F) contiennent ensemble ≤ 30 % en poids de composant V2F), par rapport à la masse totale des composants V2B) et V2F).

5. Élément viscoélastique selon l'une quelconque des revendications précédentes, dans lequel le composant V2A) est le diisocyanate d'isophorone et/ou le diisocyanate d'hexaméthylène.

6. Élément viscoélastique selon l'une quelconque des revendications précédentes, **caractérisé en ce que** le composant V2B) contient des poly(tétraméthylèneglycol)-polyétherpolyols ou consiste en ces derniers.

7. Élément viscoélastique selon l'une quelconque des revendications précédentes, **caractérisé en ce que** le composant (V1) est un prépolymère à fonction isocyanate ayant une teneur pondérale en diisocyanates aliphatiques de faible masse moléculaire ayant une masse molaire de ≥ 140 à ≤ 278 g/mole, de ≤ 1,0 % en poids, par rapport au prépolymère, pouvant être obtenu par mise en réaction de
V1A) diisocyanates aliphatiques de faible masse moléculaire ayant une masse molaire de ≥ 140 à ≤ 278 g/mole, de préférence ≥ 168 à ≤ 258 g/mole, avec
V1B) des polyoxyalkylènes di- à hexafonctionnels ayant un indice de groupes OH de ≥ 22,5 à ≤ 112 mg de KOH/g et une proportion d'oxyde d'éthylène de ≥ 50 à ≤ 100 % en moles, par rapport à la quantité totale des groupes oxyalkylène contenus ;
V1C) éventuellement des oligomères, tétracycliques ou hexacycliques, hétérocycliques, de diisocyanates aliphatiques de faible masse moléculaire ayant une masse molaire de ≥ 140 à ≤ 278 g/mole, de préférence ≥ 168 à ≤ 258 g/mole ;
V1D) éventuellement des catalyseurs ;
V1E) éventuellement des sels de métaux alcalins d'acides inorganiques faibles ;
V1F) éventuellement des tensioactifs ;
V1G) éventuellement des alcools mono- ou polyhydriques ;
V1H) les composants suivants :
V1H1) un ou plusieurs diisocyanates aliphatiques de faible masse moléculaire ayant une masse molaire de ≥ 140 à ≤ 278 g/mole, de préférence ≥ 168 à ≤ 258 g/mole et/ou polyisocyanates pouvant être préparés à partir de ceux-ci, ayant une fonctionnalité isocyanate de ≥ 2 à ≤ 6 ;
et/ou
V1H2) un ou plusieurs polyoxyalkylènes mono-fonctionnels ayant un indice de groupes OH de ≥ 10 à ≤ 250 et une proportion d'oxyde d'éthylène de ≥ 50 à ≤ 100 % en moles, par rapport à la quantité totale des groupes oxyalkylène contenus ;
et/ou
V1H3) un composant isocyanate hydrophile qui peut être obtenu par la mise en réaction de composants mentionnés en V1H1) avec des composants mentionnés en V1H2).

8. Procédé pour la préparation d'un élément viscoélastique, comprenant les étapes :
I) mélange d'un prépolymère-polyuréthane (V1) à fonction isocyanate avec au moins une dispersion de polyuréthane-urée (V2) en présence d'un milieu contenant des groupes réactifs avec des isocyanates, avec obtention d'un mélange milieu-prépolymère-polyuréthane, le milieu étant de préférence l'eau ;
II) application du mélange milieu-prépolymère-polyuréthane provenant de l'étape I) sur un support,
III) durcissement du mélange milieu-prépolymère-polyuréthane, en particulier d'un mélange eau-prépolymère-polyuréthane, provenant de l'étape II), avec obtention de l'élément viscoélastique,
IV) éventuellement enlèvement de l'élément viscoélastique d'avec le support.

9. Procédé selon la revendication 8, dans lequel le mélange dans l'étape I) s'effectue en l'espace de 3 à 90 secondes, avant l'application du mélange sur le support dans l'étape II).

10. Bouchon de protection auditive destiné à l'introduction dans une oreille, comportant un élément viscoélastique selon l'une quelconque des revendications 1 à 7 ou préparé selon l'une quelconque des revendications 8 et 9.

11. Utilisation d'un élément viscoélastique selon l'une quelconque des revendications 1 à 7, préparé conformément à un procédé selon l'une quelconque des revendications 8 et 9, pour la réduction au minimum de bruit dans un espace intérieur.

12. Ensemble destiné à la production d'un élément viscoélastique, consistant en
(V1) un prépolymère-polyuréthane à fonction isocyanate ;
(V2) une dispersion de polyuréthane-urée.

13. Ensemble selon la revendication 12, dans lequel le composant (V2) se trouve sous forme de dispersion aqueuse.

14. Ensemble selon la revendication 12 ou 13, dans lequel le composant (V1) peut être obtenu par mise en réaction d'au moins les composants
V1A) diisocyanates aliphatiques avec
V1B) des polyoxyalkylènes di- à hexafonctionnels ayant une proportion d'oxyde d'éthylène de 50 à 100 % en moles, par rapport à la quantité totale des groupes oxyalkylène contenus.

15. Ensemble selon l'une quelconque des revendications 12 à 14, dans lequel la polyuréthane-urée de la dispersion de polyuréthane-urée V2) peut être obtenue par mise en réaction d'au moins
V2A) un composant polyisocyanate aliphatique ayant une fonctionnalité isocyanate moyenne de ≥ 1,8 et ≤ 2,6 ;
V2B) un composant polymère polyéther-polyol ;
V2C) un composant extendeur de chaîne à fonction amino, comportant au moins 2 groupes amino réactifs avec des isocyanates, contenant au moins un composé C1) à fonction amino, qui ne comporte pas de groupes ioniques ou ionogènes et/ou un composé C2) à fonction amino, qui comporte des groupes ioniques ou ionogènes ;
V2D) éventuellement des composants hydrophilisants supplémentaires qui sont différents de C2),
V2E) éventuellement des composés à fonction hydroxy ayant une masse moléculaire de 62 à 399 g/moles,
V2F) éventuellement au moins un polyol polymère supplémentaire, qui est différent de V2B),
V2G) un composé qui comporte exactement un groupe réactif avec un isocyanate, ou un composé qui comporte plus d'un groupe réactif avec un isocyanate, un seul des groupes réactifs avec des isocyanates réagissant, dans les conditions réactionnelles choisies, avec les groupes isocyanate présents dans le mélange réactionnel, et éventuellement un composant polyisocyanate aliphatique ayant une fonctionnalité isocyanate moyenne de ≥ 2,6 et ≤ 4,
